# EUROPEAN PATENT APPLICATION

(11) **EP 4 740 903 A1**
(43) Date of publication of application: **13.05.2026**
(21) Application number: 24836372.3
(22) Date of filing: 05.07.2024
(51) Int. Cl.: A61C 13/00, A61C 19/04, A61B 1/24, A61B 5/00, G01B 11/00, A61C 9/00, G06T 17/00, G06T 7/00, G06T 7/11, A61B 6/51

(54) **DATA PROCESSING METHOD AND DEVICE, AND RECORDING MEDIUM**

(30) Priority: 06.07.2023 KR 20230087611; 04.07.2024 KR 20240088225
(71) Applicant: Medit Corp., Seoul 07207 (KR)
(72) Inventor: LEE, Sunghoon, Seoul 07207 (KR)
(74) Representative: MFG Patentanwälte Meyer-Wildhagen Meggle-Freund Gerhard PartG mbB
(86) International application number: PCT/KR2024/009553
(87) International publication number: WO 2025/009925

(57) **Abstract**

A method performed by an electronic device according to one embodiment of the present disclosure may include generating first data for a margin line of a target tooth included in an oral cavity, based on three-dimensional scan data for the oral cavity of a subject; generating second data for adjusting an orientation and size of a reference tooth for alignment with the target tooth, based on data for a dental library including the reference tooth corresponding to the target tooth; and generating third data for a shape of the prosthesis, based on the first data and the second data.

## Description

### TECHNICAL FIELD

The present disclosure relates to a technology for processing data. More specifically, the present disclosure relates to a technology for generating data for a shape of a prosthesis to be attached to a target tooth on the basis of three-dimensional (3D) scan data obtained by scanning an intraoral structure of a subject with a 3D scanner.

### BACKGROUND ART

Dental prosthetics are artificial replacements for one or more teeth or related tissues, and may be used to treat morphological and physiological changes in the oral cavity, which occur due to tooth loss, or to prevent diseases caused by tooth loss.

Since intraoral structures vary by individuals, fine adjustments are required to align the shape of a prosthesis to an intraoral structure. For example, detailed operations such as adjusting a gap between a tooth and the prosthesis or adjusting a height of the prosthesis to adjacent teeth are required. This prosthesis manufacturing method requires a lot of time and effort, and in particular, the proficiency of the prosthesis manufacturer has a great influence on the quality, including the fit and comfort of the prosthesis. In particular, the prosthesis attached to a tooth from which the damaged part due to decay, wear, or the like has been removed must be manufactured to accurately reflect the complex curves and shapes of the tooth.

### DISCLOSURE

### TECHNICAL PROBLEM

An embodiment of the present disclosure provides a technology for generating data for a shape of a prosthesis on the basis of three-dimensional (3D) scan data for an oral cavity of a subject.

### TECHNICAL SOLUTION

An aspect of the present disclosure may provide a method for generating data for a shape of a prosthesis on the basis of three-dimensional (3D) scan data for an oral cavity of a subject. The method according to the present disclosure may be a method performed by an electronic device comprising at least one processor and at least one memory in which instructions to be executed by the at least one processor are stored. The method according to the present disclosure may include: generating first data for a margin line of a target tooth included in an oral cavity, based on three-dimensional scan data for the oral cavity of a subject, wherein the margin line corresponds to a closed curve defining a boundary between a prosthesis to be attached to the target tooth and the target tooth; generating second data for adjusting an orientation and a size of a reference tooth for alignment with the target tooth, based on data for a dental library including the reference tooth corresponding to the target tooth; and generating third data for a shape of the prosthesis, based on the first data and the second data.

According to an embodiment, the generating the first data may include determining the target tooth among one or more teeth included in the oral cavity of the subject.

According to an embodiment, the generating the second data may include: generating fourth data for an arch curve defining a maxillary or mandibular arch including the target tooth, based on the three-dimensional scan data; and adjusting the orientation of the reference tooth, based on the fourth data.

According to an embodiment, the adjusting the orientation of the reference tooth may include: determining a first local coordinate system that defines an orientation of the target tooth with respect to the arch curve; determining a second local coordinate system that defines the orientation of the reference tooth with respect to a maxillary or mandibular arch including the reference tooth within the dental library; and adjusting the orientation of the reference tooth such that axes of the first local coordinate system and axes of the second local coordinate system are aligned.

According to an embodiment, the determining the first local coordinate system may include: determining a first direction and a second direction with respect to the arch curve at a first point among points configuring the margin line; and determining the first local coordinate system to include an axis of the first direction and an axis of the second direction.

According to an embodiment, the first direction may be a centripetal acceleration direction of the arch curve at a second point on the arch curve that is closest to the first point, and the second direction may be a tangential direction of the arch curve at the second point.

According to an embodiment, the data for the dental library may include data for a local coordinate system for each of multiple model teeth included in the dental library, and the determining the second local coordinate system may include determining a local coordinate system for the reference tooth corresponding to the target tooth among the multiple model teeth as the second local coordinate system.

According to an embodiment, the generating the second data may include adjusting the size of the reference tooth, based on a distance between the reference tooth whose orientation has been adjusted and an adjacent tooth of the target tooth.

According to an embodiment, the adjusting the size of the reference tooth may include: selecting a first point from among points constituting an outline of the reference tooth; determining a second point having a shortest distance to the first point among points constituting an outline of the adjacent tooth; determining a third point on a straight line connecting the first point and the second point; and adjusting the size of the reference tooth such that the first point is moved to a position of the third point.

According to an embodiment, the generating the third data may include: generating first mesh data for an outer face of the prosthesis; and generating second mesh data for an inner face of the prosthesis.

According to an embodiment, the generating the third data may include connecting one or more points constituting the inner face of the prosthesis and one or more points constituting the outer face of the prosthesis.

According to an embodiment, the generating the first mesh data may include: determining a normal direction with respect to a surface of the target tooth at a point configuring the margin line; determining a first offset point spaced a first offset apart from the point in the normal direction; and generating the first mesh data, based on the first offset point.

According to an embodiment, the generating the second mesh data may include: determining multiple initial points constituting a closed curve among multiple points constituting an outline of the reference tooth aligned with the target tooth; and generating third mesh data for an initial outer face of the prosthesis, based on the multiple initial points and a shape of the reference tooth.

According to an embodiment, the third mesh data may include multiple faces formed by the multiple initial points.

The method according to the present disclosure may further include: determining a moving target point located at an edge of the initial outer face of the prosthesis among the multiple initial points; determining a target face including the moving target point among the multiple faces; determining the moving target point as a control point for the target face; determining a second offset point that is spaced a second offset apart in a direction with a shortest distance from the moving target point determined as the control point with respect to the margin line; and changing the target point constituting the target face to the second offset point.

The method according to the present disclosure may further include: determining whether a size of a specific face among the multiple faces is equal to or greater than a specific value; in response to determining that the size of the specific face is equal to or greater than the specific value, dividing the specific face into multiple sub-faces; and in response to determining that the size of the specific face is less than the specific value, merging the specific face with one or more adjacent faces with respect to the specific face among the multiple faces.

An electronic device according to the present disclosure may include at least one processor and at least one memory in which instructions executed by the at least one processor are stored, wherein when the instructions are executed by the at least one processor, the at least one processor may be configured to execute the method according to the present disclosure.

A non-transitory computer-readable recording medium according to the present disclosure records instructions that, when executed by at least one processor, cause the at least one processor to perform operations, wherein the instructions may be configured to cause the at least one processor to execute the method according to the present disclosure.

### ADVANTAGEOUS EFFECTS

According to an embodiment of the present disclosure, shape data for a prosthesis may be generated on the basis of 3D scan data for an oral cavity of a subject, so that the prosthesis may be conveniently and quickly manufactured without having to directly draw a shape of the prosthesis or make many adjustments to fit the prosthesis to an intraoral cavity structure.

The effects according to the technical idea of the present disclosure are not limited to the effects described above, and other effects not mentioned may be clearly understood by those skilled in the art from the description of the specification.

### DESCRIPTION OF DRAWINGS

FIG. 1 is a diagram illustrating a system for acquiring scan data by using a 3D scanner according to an embodiment of the present disclosure.
FIG. 2A is a block diagram illustrating an electronic device and a 3D scanner according to an embodiment of the present disclosure.
FIG. 2B is a perspective diagram illustrating a 3D scanner according to an embodiment of the present disclosure.
FIG. 3A is a diagram illustrating maxillary scan data and mandibular scan data for an intraoral structure according to an embodiment of the present disclosure.
FIG. 3B is a diagram illustrating bimaxillary scan data for an intraoral structure according to an embodiment of the present disclosure.
FIG. 4A is a diagram illustrating dental notation data for a type of target teeth and prostheses according to an embodiment of the present disclosure.
FIG. 4B is a diagram illustrating 3D scan data for an oral cavity of a subject according to an embodiment of the present disclosure.
FIG. 5 is a diagram illustrating a margin line of a target tooth according to an embodiment of the present disclosure.
FIG. 6 is a diagram illustrating an arch curve defining a dental arch including a target tooth according to an embodiment of the present disclosure.
FIG. 7 is a diagram illustrating a process for determining a local coordinate system of a target tooth according to an embodiment of the present disclosure.
FIG. 8 is a diagram illustrating a dental library according to an embodiment of the present disclosure.
FIG. 9 is a diagram illustrating a local coordinate system of a reference tooth corresponding to a target tooth in a dental library according to an embodiment of the present disclosure.
FIG. 10A is a diagram illustrating a process of adjusting an orientation of a reference tooth for alignment between a target tooth and the reference tooth according to an embodiment of the present disclosure.
FIG. 10B is a diagram illustrating a result of adjusting an orientation of a reference tooth for alignment between a target tooth and the reference tooth according to an embodiment of the present disclosure.
FIG. 10C is a diagram illustrating a process of adjusting a size of a reference tooth for alignment between a target tooth and the reference tooth according to an embodiment of the present disclosure.
FIG. 10D is a diagram illustrating a result of adjusting a size of a reference tooth for alignment between a target tooth and the reference tooth according to an embodiment of the present disclosure.
FIGS. 11A and 11B are diagrams illustrating a target tooth aligned with a reference tooth according to an embodiment of the present disclosure.
FIG. 12A is a diagram illustrating a process of generating an inner face of a prosthesis according to an embodiment of the present disclosure.
FIG. 12B is a diagram illustrating a mesh for an inner face of a prosthesis according to an embodiment of the present disclosure.
FIG. 13A is a diagram illustrating a process of generating an initial outer face of a prosthesis according to an embodiment of the present disclosure.
FIG. 13B is a diagram illustrating a mesh for an initial outer face of a prosthesis according to an embodiment of the present disclosure.
FIG. 13C is a diagram illustrating a process of modifying an outer face of a prosthesis according to an embodiment of the present disclosure.
FIG. 13D is a diagram illustrating a process of adjusting a density of a mesh for an outer face of a prosthesis when modifying the outer face according to an embodiment of the present disclosure.
FIG. 13E is a diagram illustrating a modified outer face of a prosthesis according to an embodiment of the present disclosure.
FIG. 13F is a diagram illustrating a mesh for a modified outer face of a prosthesis according to an embodiment of the present disclosure.
FIG. 14 is a diagram illustrating a shape of a prosthesis according to an embodiment of the present disclosure.
FIG. 15 is a diagram illustrating a shape of a prosthesis attached to a target tooth according to an embodiment of the present disclosure.
FIG. 16 is a flowchart illustrating a method according to an embodiment of the present disclosure.

### MODE FOR INVENTION

The various embodiments described in the present disclosure are illustrated for the purpose of clarifying the technical ideas of the disclosure and are not intended to limit the disclosure to any particular embodiment. The technical ideas of the present disclosure include various modifications, equivalents, and alternatives of each embodiment of the present disclosure and include embodiments optionally combined from all or part of each embodiment. Furthermore, the scope of the technical ideas of the present disclosure is not limited to the various embodiments set forth below or to the specific description thereof.

Terms used in the present disclosure, including all technical and scientific terms, are intended to have the meanings commonly understood by those of ordinary skill in the art to which the present disclosure belongs, unless otherwise defined.

Expressions such as "comprise," "include," "may include," "provided," "may be provided," "have," "may have," and the like used in the present disclosure imply the presence of a subject feature (e.g., a function, an operation, or a component) and do not exclude the presence of other additional features. That is, the expressions should be understood as open-ended terms that imply the possibility of including other embodiments.

A singular form used in the present disclosure may include a meaning of a plurality, unless otherwise mentioned, and the same is applied to a singular expression recited in the claims.

Expressions such as "first," "second," and the like used in the present disclosure distinguish one object from another in referring to multiple objects, unless otherwise indicated in the context, and are not intended to limit the order or importance of corresponding objects.

In the present disclosure, expressions such as "A, B, and C," "A, B, or C," "A, B, and/or C," "at least one of A, B, and C," "at least one of A, B, or C," "at least one of A, B, and/or C," "at least one selected from A, B, and C," "at least one selected from A, B, or C," and "at least one selected from A, B, and/or C" may refer to each of enumerated items or any possible combination of the enumerated items. For example, "at least one selected from A and B" may refer to (1) A, (2) at least one of A, (3) B, (4) at least one of B, (5) at least one of A and at least one of B, (6) at least one of A and B, (7) at least one of B and A, (8) both A and B.

The expression "based on" or "according to" used in the present disclosure is used to describe one or more factors that influence a decision, an activity of judgment, or an operation described in a phrase or sentence including the relevant expression, and this expression does not exclude an additional factor influencing the decision, the activity of determination, or the operation.

In the present disclosure, expressions such as a certain component (e.g., a first component) being "connected to" or "coupled with" any other component (e.g., a second component) may refer to the certain component being directly connected or coupled to the other component, as well as being connected or coupled through another intervening component (e.g., a third component).

In the present disclosure, the expression "configured to" have meanings such as, depending on the context, "set to," "having an ability to," "changed to," "made to," "to do," and "able to." The expression is not limited to the meaning of "specifically designed in hardware," and for example, a processor configured to perform a specific operation may mean a special purpose computer structured through programming to perform the specific operation.

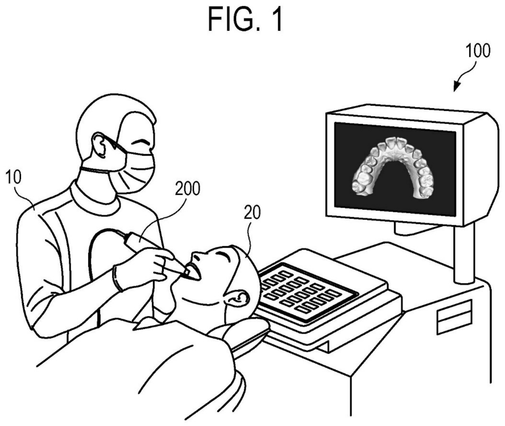

FIG. 1 is a diagram illustrating a system for acquiring scan data by using a 3D scanner 200 according to an embodiment of the present disclosure.

According to an embodiment, the 3D scanner 200 may be a dental medical device for acquiring scan data for an oral cavity of a subject 20. Here, the oral cavity of the subject 20 may be an intraoral structure of the subject 20.

For example, the 3D scanner 200 may include an intraoral scanner.

For example, a user 10 (e.g., a dentist or a dental hygienist) may acquire scan data for the oral cavity of the subject 20 (e.g., a patient) by using the 3D scanner 200.

For example, the user 10 may acquire an image of the oral cavity of the subject 20 from a diagnosis model (e.g., a plaster model or an impression model) molded after the shape of the oral cavity of the subject 20.

Hereinafter, for convenience of explanation, it is described that scan data for the oral cavity of the subject 20 is acquired by scanning the oral cavity of the subject 20, but the present disclosure is not limited thereto, and it is also possible to acquire scan data for other parts of the subject 20.

For example, the 3D scanner 200 may have a shape capable of being inserted into and drawn out of the oral cavity, and may be a handheld scanner in which the user 10 may freely adjust a scanning distance and a scanning angle.

According to an embodiment, the 3D scanner 200 may be inserted into the oral cavity of the subject 20 to scan the oral cavity in a non-contact manner, thereby acquiring scan data for the oral cavity structure.

For example, scan data for the oral cavity may represent an image including a tooth region including at least one tooth, a gingival region, an artificial structure insertable into the oral cavity, a tongue, or the like. Here, the artificial structure may include an orthodontic device including a bracket and a wire, an implant, a denture, an orthodontic auxiliary device inserted into the oral cavity, a splint, a prosthesis, and the like. For example, the prosthesis may include a crown prosthesis, an inlay prosthesis, an onlay prosthesis, and the like.

For example, the 3D scanner 200 may emit light into the oral cavity of the subject 20 using a light source (or a projector). As a specific example, the 3D scanner 200 may emit light to at least a portion of the oral cavity, such as a tooth region or a gum region, and receive light reflected from the oral cavity of the subject 20 through a camera (or an image sensor). As another example, the 3D scanner 200 may acquire scan data for the intraoral structure by scanning a diagnostic model of the oral cavity. If the diagnostic model of the oral cavity is a diagnostic model modeled after the oral cavity of the subject 20, the scan data for the diagnostic model of the oral cavity may be scan data for the intraoral structure of the subject 20. For convenience of explanation, the following description will assume a case in which scan data for the oral cavity is acquired by scanning the oral cavity of the subject 20, but the present disclosure is not limited thereto.

According to an embodiment, the 3D scanner 200 may acquire 2D scan data for the oral cavity of the object 20 on the basis of information received through the camera. Here, the 2D scan data for the oral cavity may include a 2D image of the intraoral structure.

For example, 2D scan data for the oral cavity of the subject 20 may represent a 2D image including a tooth region, a gingival region, an artificial structure, a tongue, and the like, inside the oral cavity of the subject 20.

According to an embodiment, the 2D scan data for the oral cavity acquired by the 3D scanner 200 may be transmitted to an electronic device 100 connected through a wired or wireless communication network.

For example, the electronic device 100 may include a computer device or a portable communication device. The electronic device 100 may generate 3D scan data for the oral cavity, which represents the intraoral structure in three dimensions, based on the 2D scan data for the oral cavity received from the 3D scanner 200. As a specific example, the electronic device 100 may generate 3D scan data for the oral cavity by three-dimensionally modeling the intraoral structure on the basis of the received 2D scan data for the oral cavity. For example, the electronic device 100 may generate data for the shape of a prosthesis to be attached to a target tooth in the oral cavity on the basis of the 3D scan data for the oral cavity.

According to an embodiment, the 3D scanner 200 may scan the intraoral structure of the subject 20 to acquire 2D scan data for the oral cavity, and generate 3D scan data for the oral cavity on the basis of the 2D scan data. That is, the 3D scanner 200 may generate 3D scan data for the oral cavity and transmit the same to the electronic device 100. For example, the electronic device 100 may generate data for a shape of a prosthesis to be attached to a target tooth in the oral cavity on the basis of the 3D scan data for the oral cavity.

According to an embodiment, the electronic device 100 may be communicatively connected to a cloud server or database.

For example, the electronic device 100 may transmit 2D scan data or 3D scan data for the oral cavity of the subject 20 to the cloud server or database, and the cloud server or database may store 2D scan data or 3D scan data for the intraoral structure of the subject 20 received from the electronic device 100.

For example, the electronic device 100 may receive data about a dental library from a cloud server or a database. Here, the dental library may include a maxillary dental library including one or more maxillary model teeth located in the maxilla in the oral cavity, a dental library for one or more mandibular model teeth located in the mandible in the oral cavity, and a bimaxillary library for one or more model teeth located in both jaws in the oral cavity. The bimaxillary dental library may include a maxillary dental library and a mandibular dental library.

For example, data for the maxillary teeth library may include data for a maxillary arch curve defining a dental arch of the maxilla in the oral cavity, data for a local coordinate system of each of one or more maxillary model teeth, data for an adjacent tooth of each of one or more maxillary model teeth, data for an antagonist tooth of each of one or more maxillary model teeth, and the like.

For example, the data for the maxillary arch curve may include multiple points that constitute the maxillary arch curve.

For example, the data for the local coordinate system of the maxillary model tooth may represent a local coordinate system including two or more axes representing an orientation of the maxillary model tooth based on the maxillary arch curve. For example, the data for the local coordinate system of the maxillary model tooth may represent a local coordinate system including an axis in the centripetal acceleration direction and an axis in the tangential direction at a specific point of the maxillary arch curve that is closest to multiple points configuring the shape of the maxillary model tooth. Here, the tangential direction of the maxillary arch curve may be a direction in which adjacent teeth of the maxillary model tooth are located, and the centripetal acceleration direction may be a direction in which the center of the maxillary arch is located. The direction in which the center of the maxillary arch is located may also be expressed as a buccal direction.

For example, the data for the adjacent tooth of the maxillary model tooth may represent a tooth number of an adjacent tooth, a distance between the maxillary model tooth and the adjacent tooth, or the like.

For example, the data for the antagonist tooth of the maxillary model tooth may represent a tooth number of an antagonist tooth, a distance between the maxillary model tooth and the antagonist tooth, or the like.

For example, data for the mandibular teeth library may include data for a mandibular arch curve defining the arch of the mandible within the oral cavity, data for a local coordinate system of each of one or more mandibular model teeth, data for an adjacent tooth of each of one or more mandibular model teeth, data for an antagonist tooth of each of one or more mandibular model teeth, and the like.

For example, the data for the mandibular arch curve may include multiple points that constitute the mandibular arch curve.

For example, the data for the local coordinate system of the maxillary model tooth may represent a local coordinate system including two or more axes representing an orientation of a corresponding mandibular reference based on the mandibular arch curve. For example, the data for the local coordinate system of the mandibular model tooth may represent a local coordinate system including an axis in the centripetal acceleration direction and an axis in the tangential direction at a specific point of the mandibular arch curve that is closest to multiple points configuring a shape of the mandibular model tooth. Here, the tangential direction of the mandibular arch curve may be a direction in which adjacent teeth of the mandibular model tooth are located, and the centripetal acceleration direction may be a direction in which the center of the mandibular arch is located. The direction in which the center of the mandibular arch is located may also be expressed as a buccal direction.

For example, the data for the adjacent tooth of the mandibular model tooth may represent a tooth number of an adjacent tooth, a distance between the mandibular model tooth and the adjacent tooth, or the like.

For example, the data for the antagonist tooth of the mandibular model tooth may represent a tooth number of an antagonist tooth, a distance between the mandibular model tooth and the antagonist tooth, or the like.

Meanwhile, in the present disclosure, the tooth number may be a number assigned according to the FDI system, the Palmer system, the universal numbering system, and the like.

For example, the data for the bimaxillary teeth library may include data for the maxillary teeth library and data for the mandibular teeth library.

Although the 3D scanner 200 was described above with a focus on a handheld scanner, the present disclosure may also be applied to a table scanner that is fixed to a specific location and used. That is, the method proposed in the present disclosure may also be implemented by a table scanner. The table scanner may generate 3D scan data for a diagnostic model of the oral cavity by scanning the diagnostic model of the oral cavity. Since the light source (or projector) and camera of the table scanner are fixed, the user 10 may scan the diagnostic model of the oral cavity while moving the diagnostic model of the oral cavity.

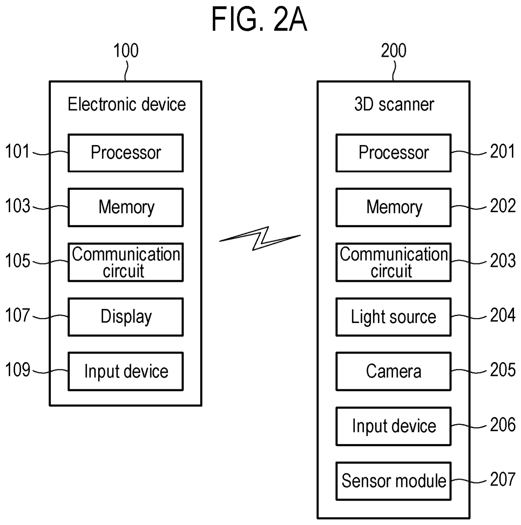

FIG. 2A is a block view illustrating an electronic device 100 and a 3D scanner 200 according to an embodiment of the present disclosure.

According to an embodiment, the electronic device 100 and the 3D scanner 200 may be communicatively connected to each other through a wired or wireless communication network, and may transmit and receive various data to and from each other.

According to an embodiment, the 3D scanner 200 may include at least one of a processor 201, a memory 202, a communication circuit 203, a light source 204, a camera 205, an input device 206, and a sensor module (sensor) 207. At least one of components included in the 3D scanner 200 may be omitted or another component may be added to the 3D scanner 200. Additionally or alternatively, some of the components may be integrated and implemented, or implemented as a singular or plural entities. At least some of the components in the 3D scanner 200 may be connected to each other through a bus, a general purpose input/output (GPIO), a serial peripheral interface (SPI) or a mobile industry processor interface (MIPI), and transmit and receive data and/or a signal.

According to an embodiment, the processor 201 of the 3D scanner 200 corresponds to a component capable of performing calculation or data processing for control or communication of each component of the 3D scanner 200 and may be operatively connected to the components of the 3D scanner 200. The processor 201 may load a command or data received from other components of the 3D scanner 200 into the memory 202, process the command or data stored in the memory 202, and store result data.

According to an embodiment, the memory 202 of the 3D scanner 200 may store at least one instruction for the operation of the processor 201.

According to an embodiment, the communication circuit 203 of the 3D scanner 200 may establish a wired or wireless communication channel with an external device including the electronic device 100 and transmit and receive various data to and from the external device.

For example, the communication circuit 203 may include at least one port for connection with the external device through a wired cable to communicate with the external device by wire. In this case, the communication circuit 203 may perform communication with the external device connected via a wire through at least one port.

For example, the communication circuit 203 may include a cellular communication module and may be configured to be connected to a cellular network (e.g., 3G, LTE, 5G, Wibro or WiMAX).

For example, the communication circuit 203 may include a short-range communication module to transmit and receive data to and from the external device including the electronic device 100 using short-range communication (e.g., Wi-Fi, BLE (Bluetooth, Bluetooth Low Energy), or UWB).

For example, the communication circuit 203 may include a non-contact communication module for non-contact communication. As a specific example, the non-contact communication may include at least one non-contact type proximity communication technology such as near field communication (NFC) communication, radio frequency identification (RFID) communication, or magnetic secure transmission (MST) communication.

According to an embodiment, the light source 204 of the 3D scanner 200 may emit light toward the inside of the oral cavity of the subject 20.

For example, the light emitted from the light source 204 may be structured light having a predetermined pattern. As a specific example, the predetermined pattern may be a stripe pattern in which straight lines of different colors appear continuously. The pattern of the structured light may be generated using a pattern mask or a digital micro-mirror device (DMD), but is not limited thereto.

According to an embodiment, the camera 205 of the 3D scanner 200 may acquire 2D scan data for the intraoral structure of the subject 20 by receiving reflected light reflected by the oral cavity of the subject 20.

For example, the camera 205 may include a left camera corresponding to the left eye field of view and a right camera corresponding to the right eye field of view to construct 3D scan data according to an optical triangulation method.

For example, the camera 205 may include at least one image sensor such as a CCD sensor or a CMOS sensor.

According to an embodiment, the input device 206 of the 3D scanner 200 may receive a user input for controlling the three-dimensional scanner 200.

For example, the input device 206 may include at least one of a button for receiving a push operation of the user 10, a touch panel for detecting a touch of the user 10, or a voice recognition device including a microphone.

For example, the user 10 may control scanning start or stop by using the input device 206.

According to an embodiment, the sensor module 207 of the three-dimensional scanner 200 may detect an operation state of the three-dimensional scanner 200 or an external environmental state (e.g., a user's motion), and generate an electrical signal corresponding to the detected state.

For example, the sensor module 207 may include at least one of a gyro sensor, an acceleration sensor, a gesture sensor, a proximity sensor, or an infrared sensor.

For example, the user 10 may control scanning start or stop by using the sensor module 207. As a specific example, when the user 10 holds the 3D scanner 200 in his or her hand and moves the same, the 3D scanner 200 may control, when an angular velocity measured through the sensor module 207 exceeds a predetermined threshold value, the processor 201 to start a scanning operation.

According to an embodiment, in response to receiving a user input to initiate a scan through the input device 206 of the 3D scanner 200 or the input device 109 of the electronic device 100, the 3D scanner 200 may initiate a scan.

According to an embodiment, the 3D scanner 200 may start scanning on the basis of processing by the processor 201 of the 3D scanner 200 or the processor 101 of the electronic device 100.

According to an embodiment, when the user 10 scans the intraoral structure of the subject 20 by using the 3D scanner 200, the 3D scanner 200 may generate 2D scan data for the oral cavity of the subject 20 and transmit the 2D scan data for the oral cavity of the subject 20 to the electronic device 100 in real time.

For example, the electronic device 100 may display a 2D image of the oral cavity represented by the received 2D scan data for the intraoral structure of the subject 20 through a display 107.

For example, the electronic device 100 may generate 3D scan data for the oral cavity of the subject 20 on the basis of the 2D scan data for the oral cavity of the subject 20. In addition, the electronic device 100 may display a 3D image of the oral cavity represented by the received 3D scan data for the intraoral structure of the subject 20 through the display 107. The electronic device 100 may also display the process of generating the 3D scan data for the oral cavity in real time through the display 107.

For example, the electronic device 100 may generate data for the shape of a prosthesis to be attached to a target tooth in the oral cavity on the basis of the 3D scan data for the oral cavity of the subject 20. Here, the data for the shape of the prosthesis may be referred to as an image of the shape of the prosthesis. In addition, the electronic device 100 may display an image of the shape of the prosthesis through the display 107. The electronic device 100 may also display a process of generating data for the shape of the prosthesis in real time through the display 107.

According to an embodiment, the electronic device 100 may include at least one of one or more processors 101, one or more memories 103, a communication circuit 105, a display 107, or an input device 109. At least one of components included in the electronic device 100 may be omitted or another component may be added to the electronic device 100. Additionally or alternatively, some of the components may be integrated and implemented, or implemented as singular or plural entities. At least some of the components within the electronic device 100 may be connected to each other via a bus, GPIO, SPI, MIPI, or the like, and exchange signals or data with each other.

According to an embodiment, the at least one processor 101 of the electronic device 100 may correspond to a component capable of performing calculation or data processing for control and communication of each component of the electronic device 100.

For example, the one or more processors 101 may be operatively connected to components of the electronic device 100. The one or more processors 101 may load a command or data received from other components of the electronic device 100 into the one or more memories 103, process the command or data stored in the one or more memories 103, and store result data.

According to an embodiment, the at least one memory 103 of the electronic device 100 may store at least one instruction for an operation of the at least one processor 101.

For example, the one or more memories 103 may store data (e.g., 2D scan data or 3D scan data for the oral cavity) received from the 3D scanner 200.

According to an embodiment, the communication circuit 105 of the electronic device 100 may establish a wired or wireless communication channel with an external device including at least one of the 3D scanner 200, a cloud server, a database, or the like, and may transmit and receive various data to and from the external device.

For example, the communication circuit 105 may include at least one port for connection with the external device through a wired cable to communicate with the external device by wire. In this case, the communication circuit 105 may perform communication with the external device connected via a wire through at least one port.

For example, the communication circuit 105 may include a cellular communication module and may be configured to be connected to a cellular network (e.g., 3G, LTE, 5G, Wibro, or WiMAX).

For example, the communication circuit 105 may include a short-range communication module to transmit and receive data to and from an external device including at least one of the 3D scanner 200, a cloud server, a database, or the like, by using short-range communication (e.g., Wi-Fi, BLE, UWB).

For example, the communication circuit 105 may include a non-contact communication module for non-contact communication. As a specific example, the non-contact communication may include at least one non-contact type proximity communication technology such as NFC communication, RFID communication, or MST communication.

According to an embodiment, the display 107 of the electronic device 100 may display various screens on the basis of control of the processor 101.

For example, the processor 101 of the electronic device 100 may control components of the electronic device 100 to display 2D scan data or 3D scan data for the oral cavity of the subject 20 through the display 107.

For example, the processor 101 may control components of the electronic device 100 to display data for a shape of a prosthesis to be attached to a target tooth in the oral cavity of the subject 20 through the display 107.

For example, a specific application execution screen may be displayed on the display 107 of the electronic device 100, and 2D scan data or 3D scan data for the oral cavity may be displayed on the execution screen. In addition, data for the shape of a prosthesis may be displayed on the execution screen. Here, a web browser or application for executing a specific application may be installed on the electronic device 100. The user 10 may edit, save, and delete 2D scan data or 3D scan data for the oral cavity displayed on the display 107 by using the input device 109. The user 10 may edit, save, and delete data necessary to generate data for the shape of the prosthesis displayed on the display 107 by using the input device 109. The user 10 may edit, save, and delete data for the shape of the prosthesis displayed on the display 107 by using the input device 109.

According to an embodiment, the input device 109 of the electronic device 100 may receive a command or data to be used for a component (e.g., the at least one processor 101) of the electronic device 100 from the outside (e.g., the user 10) of the electronic device 100.

For example, the input device 109 may be combined with the display 107 and implemented in a form of a touch sensor panel capable of recognizing contact or proximity of various external objects.

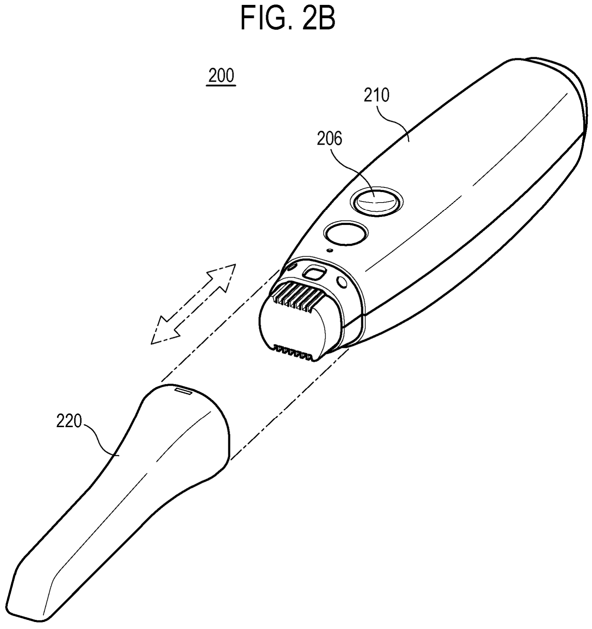

FIG. 2B is a perspective view illustrating a 3D scanner 200 according to an embodiment of the present disclosure.

According to an embodiment, the 3D scanner 200 may include a body 210 and a probe tip 220.

For example, the body 210 may have a shape that is easy for the user 10 to hold by hand.

For example, the probe tip 220 may have a shape that is easy to be inserted into and drawn out from the oral cavity of the subject 20.

For example, the body 210 may be coupled to and separated from the probe tip 220.

For example, the body 210 may have the components of the 3D scanner 200 described in FIG. 2A disposed therein.

For example, an opening may be formed at one end of the body 210 so that light output from the light source 204 may be emitted into the oral cavity of the subject 20. The light emitted through the opening may be reflected by the intraoral structure of the subject 20 and introduced again through the opening. The reflected light entering through the opening may be captured by the camera 205 to generate 2D scan data for the oral cavity of the subject 20.

For example, the user 10 may start scanning by using the input device 206 (e.g., a button) of the 3D scanner 200. As a specific example, when the user 10 touches or presses the input device 206, light may be emitted from the light source 204 to the subject 20.

According to an embodiment, the user 10 may scan the intraoral structure of the subject 20 while moving the 3D scanner 200, and the 3D scanner 200 may acquire 2D scan data for the oral cavity of the subject 20.

According to an embodiment, the user may scan a diagnostic model of the inside of the oral cavity of the subject 20 while moving the 3D scanner 200, and may acquire 2D scan data for the diagnostic model in the process. Here, the 2D scan data for the diagnostic model may represent an image of the shape of the intraoral structure.

For example, the 2D scan data for the oral cavity may represent a 2D image for an area including the front teeth of the subject 20, a 2D image for an area including the molars of the subject 20, and the like.

For example, the 3D scanner 200 may transmit the acquired 2D scan data to the electronic device 100. In this case, the electronic device 100 may generate 3D scan data for the oral cavity on the basis of the received 2D scan data for the oral cavity. Here, the 3D scan data for the oral cavity may include at least one of maxillary scan data, mandibular scan data, or bimaxillary (occlusion) scan data for the oral cavity.

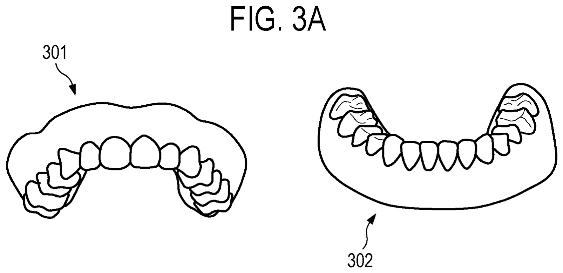

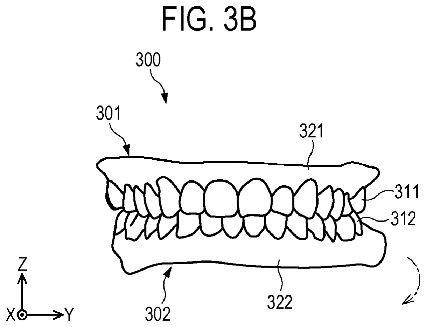

FIG. 3A is a diagram illustrating maxillary scan data 301 and mandibular scan data 302 of an intraoral structure according to an embodiment of the present disclosure. FIG. 3B is a diagram illustrating bimaxillary scan data 300 of an intraoral structure according to an embodiment of the present disclosure.

According to an embodiment, the 3D scan data for the oral cavity of the subject 20 may include at least one of maxillary scan data 301, mandibular scan data 302, or bimaxillary scan data 300. Here, the oral cavity of the subject 20 may be an intraoral structure of the subject 20.

According to an embodiment, the maxillary scan data 301 may be 3D scan data for the maxilla generated on the basis of 2D scan data acquired as a result of scanning the maxilla within the oral cavity.

For example, the maxillary scan data 301 may represent a tooth region 311 and a gingival region 321 located on the maxilla.

For example, on the basis of the maxillary scan data 301, data for the shape of a prosthesis to be attached to a target tooth located in the maxilla in the oral cavity may be generated.

According to an embodiment, the mandibular scan data 302 may be 3D scan data for the mandible generated on the basis of 2D scan data acquired as a result of scanning the mandible within the oral cavity.

For example, the mandibular scan data 302 may represent a tooth region 312 and a gingival region 322 within the mandible.

For example, on the basis of the mandibular scan data 302, data for the shape of a prosthesis to be attached to a target tooth located in the mandible in the oral cavity may be generated.

According to an embodiment, the bimaxillary scan data 300 may be 3D scan data for both jaws generated on the basis of 2D scan data acquired as a result of scanning the maxilla and the mandible in the oral cavity. Alternatively, the bimaxillary scan data 300 may be generated on the basis of the maxillary scan data 301 and the mandibular scan data 302. That is, the bimaxillary scan data 300 may be scan data representing an image generated by synthesizing an image represented by the maxillary scan data 301 and an image represented by the mandibular scan data 302.

For example, on the basis of the bimaxillary scan data 300, data for the shape of a maxillary prosthesis to be attached to a target tooth located on the maxilla within the oral cavity or data for the shape of a mandibular prosthesis to be attached to a target tooth located on the mandible within the oral cavity may be generated.

Hereinafter, the description of the embodiment proposed in the present disclosure will continue by assuming a case where data for the shape of a prosthesis to be attached to a target tooth located in the mandible in the oral cavity is generated on the basis of mandibular scan data 302. Meanwhile, this is only for convenience of explanation, and the present disclosure is not limited thereto. That is, the description below may be equally applied even when data for the shape of a prosthesis to be attached to a target tooth located in the maxilla in the oral cavity is generated on the basis of the maxillary scan data 301. In addition, the description below may be equally applied even when data for the shape of a prosthesis to be attached to a target tooth located in the maxilla in the oral cavity and data for the shape of a prosthesis to be attached to a target tooth located in the mandible in the oral cavity are generated on the basis of the bimaxillary scan data 300.

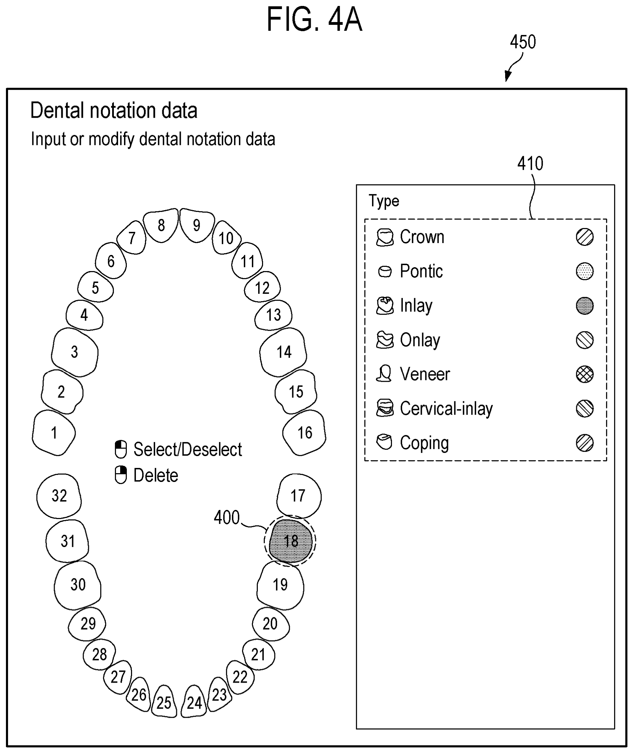

FIG. 4A is a diagram illustrating dental notation data 450 for a target tooth 400 and a type 410 of a prosthesis according to an embodiment of the present disclosure.

According to an embodiment, the dental notation data 450 may represent one or more teeth included in the oral cavity of the subject 20. For example, the dental notation data 450 may represent a tooth number of each of one or more teeth within the oral cavity of the subject 20.

According to an embodiment, the dental notation data 450 may represent a target tooth 400 among one or more teeth included in the oral cavity of the subject 20 and a type 410 of a prosthesis to be attached to the target tooth 400. For example, the prosthesis to be attached to the target tooth 400 may be a crown prosthesis, a pontic prosthesis, an inlay prosthesis, an onlay prosthesis, a veneer prosthesis, a cervical-inlay prosthesis, a coping prosthesis, or the like.

According to an embodiment, the electronic device 100 may display dental notation data 450 representing one or more teeth included in the oral cavity of the subject 20 through the display 107.

For example, a first user interface for selecting the target tooth 400 from among one or more teeth included in the oral cavity of a subject 20 indicated by dental notation data 450 or a second user interface for selecting a type 410 of a prosthesis to be attached to the target tooth 400 may be displayed on the display 107 of the electronic device 100. Here, the user interface may include basic elements for displaying specific information, such as an image or text, through the display 107, and elements for receiving input from the user 10, such as buttons that may be configured by utilizing these basic elements, through the input device 109.

According to an embodiment, the electronic device 100 may receive a selection input for the target tooth 400 from among one or more teeth included in the oral cavity of the subject 20 from the user 10 through the input device 109. For example, the user 10 may select the first user interface for selecting the target tooth 400 from among one or more teeth in the oral cavity of the subject 20 displayed on the display 107 of the electronic device 100 through the input device 109 of the electronic device 100. The electronic device 100 may determine the tooth corresponding to the first user interface selected by the user 10 as the target tooth 400. Subsequently, the electronic device 100 may update the dental notation data 450 to represent the target tooth 400 and display the updated dental notation data 450 on the display 107. Through this, the user 10 may conveniently configure the target tooth 400, such as adding or modifying the target tooth 400.

According to an embodiment, the electronic device 100 may receive a selection input for the type 410 of the prosthesis to be attached to the target tooth 400 from the user 10 through the input device 109. For example, the user 10 may select a second user interface for selecting the type 410 of the prosthesis to be attached to the target tooth 400 from among the types of prostheses displayed on the display 107 of the electronic device 100 through the input device 109 of the electronic device 100. The electronic device 100 may determine the type of prosthesis corresponding to the second user interface selected by the user 10 as the type 410 of the prosthesis to be attached to the target tooth 400. Subsequently, the electronic device 100 may update the dental notation data 450 to indicate the type 410 of the prosthesis to be attached to the target tooth 400, and display the updated dental notation data 450 on the display 107. For example, the type 410 of the prosthesis may be indicated in a specific shape (color or pattern) for the target tooth 400. Through this, the user 10 may conveniently configure the type 410 of the prosthesis to be attached to the target tooth 400.

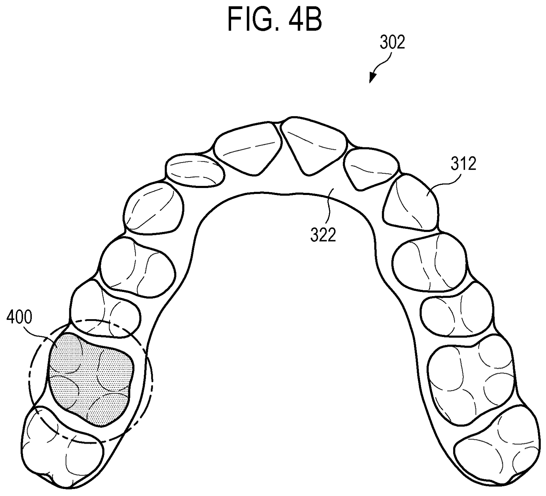

FIG. 4B is a diagram illustrating 3D scan data 302 of an oral cavity of a subject 20 according to an embodiment of the present disclosure.

According to an embodiment, the 3D scan data 302 may represent at least a portion of a tooth region 312 or a gingival region 322 located in the mandible within the oral cavity.

According to an embodiment, the tooth region 312 may include one or more teeth located in the mandible within the oral cavity.

For example, some of the teeth among one or more teeth in the tooth region 312 may be selected as target teeth 400 to which a prosthesis is to be attached. For example, the electronic device 100 may receive a selection input for a target tooth 400 among one or more teeth in the tooth region 312 from the user 10 through the input device 109. In addition, the electronic device 100 may receive a selection input for the type of the prosthesis to be attached to the target tooth 400 from the user 10 through the input device 109. This may refer to the description of FIG. 4A.

In an embodiment, the gingival region 322 may include tissue covering one or more teeth and the surrounding bone within the tooth region 312.

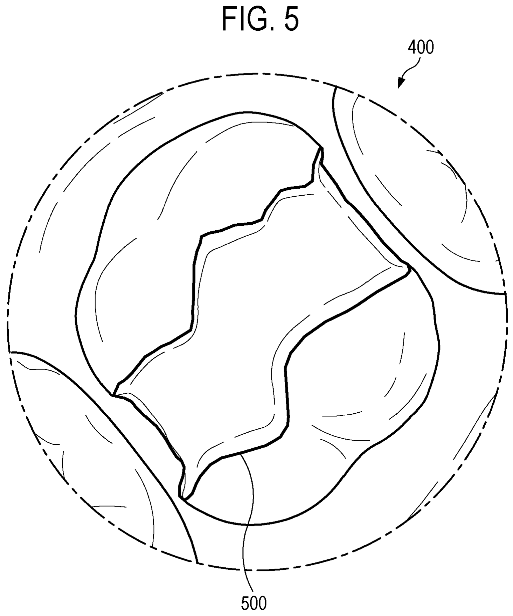

FIG. 5 is a diagram illustrating a margin line 500 of a target tooth 400 according to an embodiment of the present disclosure.

According to an embodiment, the electronic device 100 may generate data for the margin line 500 of the target tooth 400 included in the oral cavity of the subject 20 on the basis of 3D scan data 302. Here, the margin line 500 may be a closed curve defining a boundary between a prosthesis to be attached to the target tooth 400 and the target tooth 400. For example, the data for the margin line 500 may include multiple points configuring the margin line 500. Meanwhile, an operation of generating data for the margin line 500 of the target tooth 400 may also be expressed as an operation of generating the margin line 500.

For example, the electronic device 100 may determine a specific point among multiple points configuring the shape of the target tooth 400 as an initial search point. For example, the electronic device 100 may receive a selection input for a specific point among multiple points configuring the shape of the target tooth 400 from the user 10 through the input device 109, and determine the specific point as an initial search point.

For example, the electronic device 100 may determine one or more adjacent points spaced a predetermined distance apart from the initial search point among multiple points configuring the shape of the target tooth 400. Thereafter, the electronic device 100 may calculate a curvature at each of the one or more adjacent points, and determine a direction from the initial search point to the adjacent point having the largest absolute value of curvature as an initial search direction.

For example, the electronic device 100 may determine a first search point spaced a predetermined distance apart from an initial search point in the search direction among multiple points configuring the shape of the target tooth 400. The electronic device 100 may determine one or more adjacent points spaced a predetermined distance apart from the first search point among multiple points configuring the shape of the target tooth 400. Thereafter, the electronic device 100 may calculate a curvature at each of the one or more adjacent points with respect to the first search point, and determine a direction from the first search point to the adjacent point having the largest absolute value of curvature as a first search direction.

For example, the electronic device 100 may determine a second search point spaced a predetermined distance apart from the first search point in the first search direction among multiple points configuring the shape of the target tooth 400. The electronic device 100 may repeatedly perform the operation of determining the search points and the search direction until the determined search points may configure one closed curve. The electronic device 100 may determine the search points for one path to generate the margin line 500. Alternatively, the electronic device 100 may determine the search points for each of two paths having different initial search directions, and merge the two paths so that the search points for the two paths may configure one closed curve to generate the margin line 500.

For example, the electronic device 100 may receive a selection input for the margin line 500 of the target tooth 400 from the user 10 through the input device 109. For example, the selection input for the margin line 500 may include multiple points configuring the margin line 500. The electronic device 100 may generate the margin line 500 on the basis of the selection input for the margin line 500.

Meanwhile, the present disclosure is not limited to the above-described examples, and the electronic device 100 may generate the margin line 500 of the target tooth 400 according to various methods.

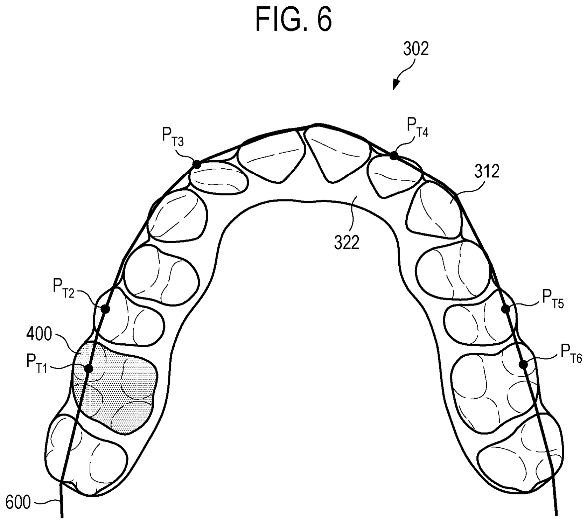

FIG. 6 is a diagram illustrating an arch curve 600 defining a dental arch including a target tooth 400 according to an embodiment of the present disclosure.

According to an embodiment, the electronic device 100 may generate data for the arch curve 600 defining a dental arch including the target tooth 400 on the basis of 3D scan data 302 of the oral cavity of the subject 20. Meanwhile, since the 3D scan data 302 is assumed to be mandibular scan data 302, the arch curve 600 is a curve defining an arch of the mandible including the target tooth 400. For example, the data for the arch curve 600 may include multiple points configuring the arch curve 600. Meanwhile, an operation of generating data for the arch curve 600 may also be expressed as an operation of generating the arch curve 600.

For example, the electronic device 100 may determine a specific point among multiple points configuring the shape of a tooth for each of one or more teeth in the tooth region 312. Here, the specific point may be a cusp point corresponding to the point having the highest height. For example, the electronic device 100 may determine a center for one or more specific points determined for each of one or more teeth. For example, the electronic device 100 may determine a plane passing through a center for the one or more specific points and having a normal vector of an occlusal face for the tooth region 312. For example, the electronic device 100 may project the one or more specific points onto the plane. For example, the electronic device 100 may generate a 2D convex hull for the one or more specific points projected onto the plane, and determine one or more points located at the outermost portion of the 2D convex hull. Here, the 2D convex hull may be expressed as a sample curve. Meanwhile, since the points on the 2D convex hull projected onto the plane may have irregular intervals, the electronic device 100 may interpolate the points so that the intervals of the points on the 2D convex hull are constant so as to generate a resample curve. For example, the electronic device 100 may adjust the points on a molar side on the resample curve to modify the resample curve to become a convex curve. For example, the electronic device 100 may project a portion of one or more teeth onto the plane and then generate a box for the teeth projected onto the plane. For example, the electronic device 100 may extend an end point of the resample curve in a tangential direction from the end point until the box generated for the teeth projected onto the plane and the resample curve meet. When this process is completed, the electronic device 100 may project multiple points on the resample curve back onto the tooth region 312. The multiple points on the resample curve projected onto the tooth region 312 may be outline points P_{T1}, P_{T2}, P_{T3}, P_{T4}, P_{T5}, and P_{T6} of some of the teeth included in the tooth region 312. Accordingly, the arch curve 600 may be generated so as to pass through the outlines of some of the teeth included in the tooth region 312. Here, the arch curve 600 passing through the outlines of some of the teeth included in the tooth region 312 may be expressed as the outlines of some of the teeth with the arch curve 600 being circumscribed. For example, the arch curve 600 may be generated so that the arch curve 600 comes into contact with the outline points P_{T1}, P_{T2}, P_{T3}, P_{T4}, P_{T5}, and P_{T6} of some of the teeth included in the tooth region 312.

Meanwhile, the present disclosure is not limited to the above-described examples, and the electronic device 100 may generate the arch curve 600 according to various methods.

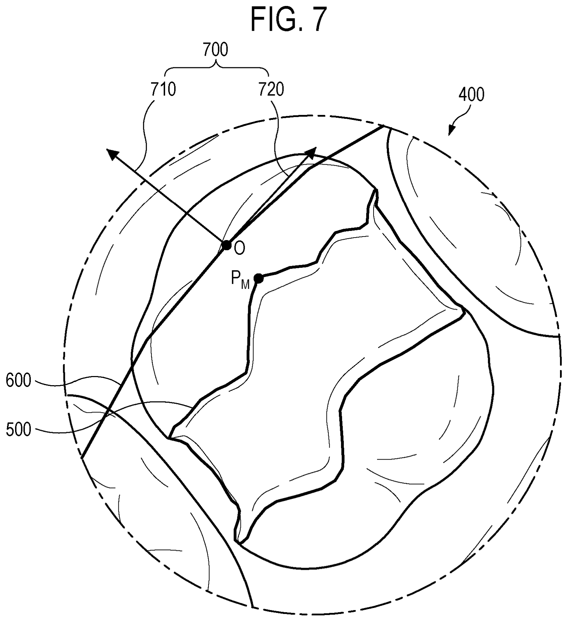

FIG. 7 is a diagram illustrating a process for determining a local coordinate system 700 of a target tooth 400 according to an embodiment of the present disclosure.

According to an embodiment, the electronic device 100 may determine the local coordinate system 700 that defines an orientation of the target tooth 400 with respect to the arch curve 600. The orientation of the target tooth 400 may be defined by two or more axes included in the local coordinate system 700.

For example, when the arch curve 600 passes through a specific point among multiple points configuring the target tooth 400, the electronic device 100 may determine a first direction 710 and a second direction 720 for the arch curve 600 at the specific point. For example, the first direction 710 may be a centripetal acceleration direction of the arch curve 600 at the specific point. For example, the second direction 720 may be a tangential direction of the arch curve 600 at the specific point. Thereafter, the electronic device 100 may determine the local coordinate system 700 to include an axis of the first direction 710 and an axis of the second direction 720. Accordingly, the local coordinate system 700 may include an axis of the first direction 710 and an axis of the second direction 720.

For example, if the arch curve 600 does not pass through any one of the multiple points configuring the target tooth 400, the electronic device 100 may determine the first direction 710 and the second direction 720 for the arch curve 600 at a specific point on the arch curve 600 that is closest to the multiple points configuring the target tooth 400. For example, the first direction 710 may be a centripetal acceleration direction of the arch curve 600 at the specific point. For example, the second direction 720 may be a tangential direction of the arch curve 600 at the specific point. Thereafter, the electronic device 100 may determine the local coordinate system 700 to include an axis of the first direction 710 and an axis of the second direction 720. Accordingly, the local coordinate system 700 may include an axis of the first direction 710 and an axis of the second direction 720.

For example, if the arch curve 600 does not pass through any one of the multiple points configuring the target tooth 40, the electronic device 100 may determine the first direction 710 and the second direction 720 for the arch curve 600 at a specific point P_{M} among the multiple points configuring the margin line. As a specific example, the electronic device 100 may determine the first direction 710 and the second direction 720 for the arch curve 600 at a specific point on the arch curve 600 that is closest to the multiple points P_{M} configuring the margin line 500. For example, the first direction 710 may be a centripetal acceleration direction of the arch curve 600 at the specific point. For example, the second direction 720 may be a tangential direction of the arch curve 600 at the specific point. Thereafter, the electronic device 100 may determine the local coordinate system 700 to include an axis of the first direction 710 and an axis of the second direction 720. Accordingly, the local coordinate system 700 may include an axis of the first direction 710 and an axis of the second direction 720.

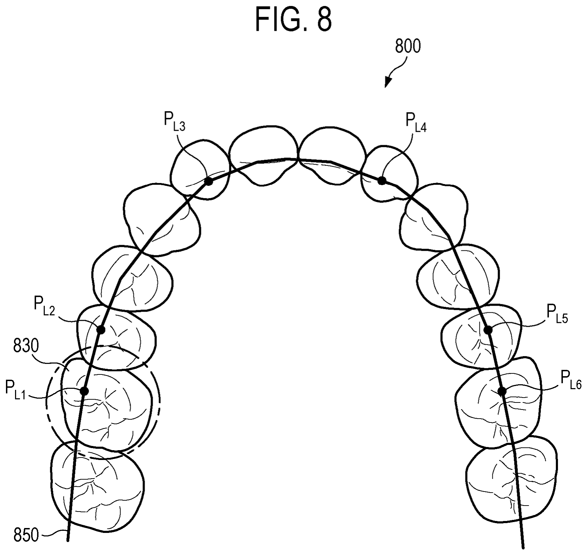

FIG. 8 is a diagram illustrating a dental library 800 according to an embodiment of the present disclosure.

According to an embodiment, the dental library 800 may include a maxillary dental library, a mandibular dental library, a bimaxillary dental library, and the like. In the following, it is assumed that the dental library 800 includes the mandibular dental library. In addition, this assumption is for convenience of explanation and the present disclosure is not limited thereto. Even when the dental library 800 includes the maxillary dental library or the bimaxillary dental library, the description below may be equally applied.

According to an embodiment, the dental library 800 may include an arch curve 850 defining the arch of the mandible within the oral cavity.

For example, the arch curve 850 may be a curve passing through the outlines of some of model teeth located in the mandible. For example, the arch curve 850 may pass through the outline points P_{L1}, P_{L2}, P_{L3}, P_{L4}, P_{L5}, and P_{L6} of some of the model teeth located in the mandible.

For example, the dental library 800 may include a local coordinate system for each of one or more model teeth. An orientation of the model tooth with respect to the arch curve 850 may be defined by two or more axes included in the local coordinate system of the model tooth.

For example, the dental library 800 may include tooth numbers of adjacent teeth of the model tooth and a distance between the model tooth and the adjacent teeth.

For example, the dental library 800 may include a tooth number of an antagonist tooth of the model tooth and a distance between the model tooth and the antagonist tooth.

According to an embodiment, the dental library 800 may include a reference tooth 830 corresponding to the target tooth 400 among one or more model teeth. For example, the reference tooth 830 may have the same tooth number as the target tooth 400.

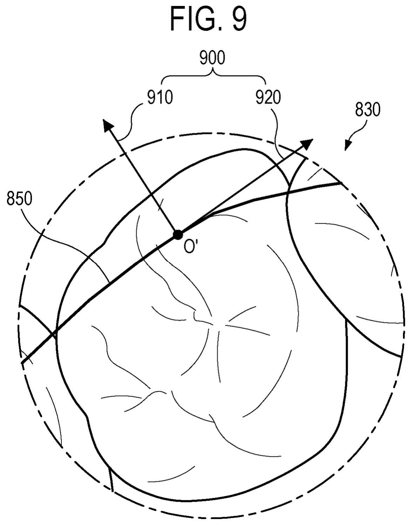

FIG. 9 is a diagram illustrating a local coordinate system 900 of a reference tooth 830 corresponding to a target tooth 400 in a dental library 800 according to an embodiment of the present disclosure.

According to an embodiment, the dental library 800 may include the local coordinate system 900 of the reference tooth 830.

For example, when the arch curve 850 passes through a specific point among multiple points configuring the reference tooth 830, a third direction 910 and a fourth direction 920 for the arch curve 850 at the specific point may be determined. For example, the third direction 910 may be a centripetal acceleration direction of the arch curve 850 at the specific point. For example, the fourth direction 920 may be a tangential direction of the arch curve 850 at the specific point. The local coordinate system 900 may include an axis of the third direction 910 and an axis of the fourth direction 920.

For example, if the arch curve 850 does not pass through any one of the multiple points configuring the reference tooth 830, the third direction 910 and the fourth direction 920 for the arch curve 850 at a specific point on the arch curve 850 that is closest to the multiple points configuring the reference tooth 830 may be determined. For example, the third direction 910 may be a centripetal acceleration direction of the arch curve 850 at the specific point. For example, the fourth direction 920 may be a tangential direction of the arch curve 850 at the specific point. The local coordinate system 900 may include an axis of the third direction 910 and an axis of the fourth direction 920.

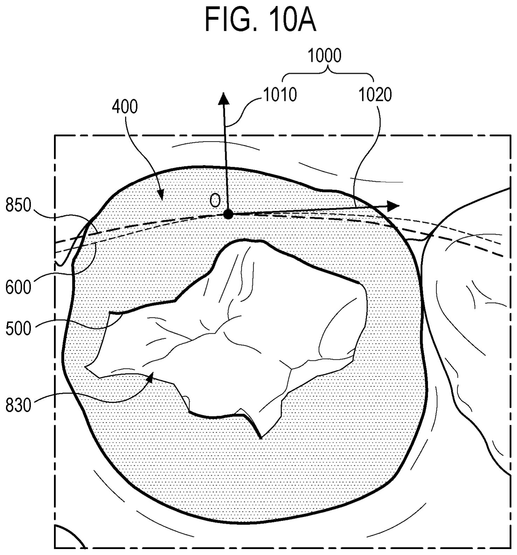

FIG. 10A is a diagram illustrating a process of adjusting an orientation of a reference tooth 830 for alignment between a target tooth 400 and the reference tooth 830 according to an embodiment of the present disclosure. Since the occlusion state or arrangement of teeth may differ from person to person, an orientation of the target tooth 400 with respect to the arch curve 600 may be different from an orientation of the reference tooth 830 with respect to the arch curve 850. If the orientation of the reference tooth 830 is different from that of the target tooth 400, the reference tooth 830 may not be accurately aligned with the target tooth 400, which may cause an error in generating the shape of a prosthesis. Therefore, the electronic device 100 may adjust the orientation of the reference tooth 830 for alignment between the target tooth 400 and the reference tooth 830.

According to an embodiment, the electronic device 100 may adjust the orientation of the reference tooth 830 on the basis of the arch curve 600.

For example, the electronic device 100 may adjust the orientation of the reference tooth 830 on the basis of the local coordinate system 700 defining the orientation of the target tooth 400 with respect to the arch curve 600 and the local coordinate system 900 defining the orientation of the reference tooth 830 with respect to the arch curve 830.

For example, the electronic device 100 may adjust the orientation of the reference tooth 830 so that the axes 710 and 720 of the local coordinate system 700 of the target tooth 400 and the axes 910 and 920 of the local coordinate system 900 of the reference tooth 830 are aligned. To this end, the electronic device 100 may adjust the orientation of the reference tooth 830 by rotating the reference tooth 830 so that the axis of the third direction 910 of the local coordinate system 900 is aligned with the axis of the first direction 710 of the local coordinate system 700, and the axis of the fourth direction 920 of the local coordinate system 900 is aligned with the axis of the second direction 720 of the local coordinate system 700. Accordingly, the target tooth 400 and the reference tooth 830 may share a common local coordinate system 1000 including a common axis in a first direction 1010 and a common axis in a second direction 1020.

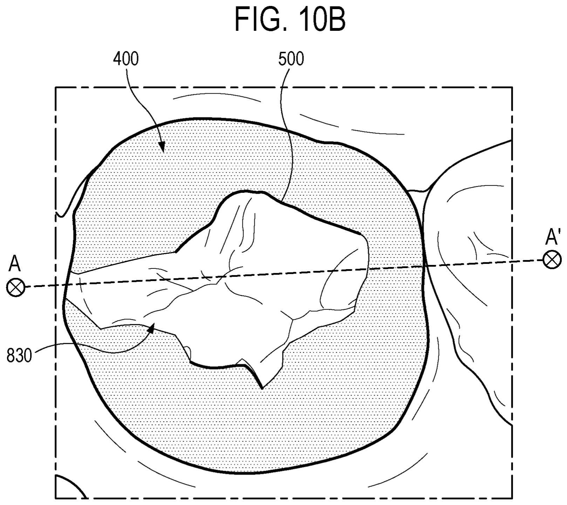

FIG. 10B is a diagram illustrating a result of adjusting the orientation of the reference tooth 830 for alignment between a target tooth 400 and the reference tooth 830 according to an embodiment of the present disclosure.

According to the result of adjusting the orientation of the reference tooth 830 described in FIG. 10A, the target tooth 400 and the reference tooth 830 may share a common local coordinate system 1000. On the basis of this common local coordinate system 1000, the relationship (relative position, distance, or the like) between points on the margin line 500 of the target tooth 400 and points configuring the shape of the reference tooth 830 may be defined, and the shape of the prosthesis may be generated on the basis of the shape of the reference tooth 830. Meanwhile, since the size of teeth is different for each person, in order to use the shape of the reference tooth 830 to generate the shape of the prosthesis, even when the orientation of the reference tooth 830 is adjusted, it is necessary to adjust the size of the reference tooth 830 to be similar to that of the target tooth 400. For example, if the reference tooth 830 is much smaller than the target tooth 400, at least a portion of the shape of a prosthesis to be attached to the target tooth 400 may not be determined on the basis of the reference tooth 830, or the shape of a dental prosthesis may be distorted according to the shape of the reference tooth 830 that does not fit the size. Alternatively, if the reference tooth 830 is much larger than the target tooth 400, the shape of the prosthesis to be attached to the target tooth 400 may be distorted according to the shape of the reference tooth 830 that does not fit the size. Referring to FIG. 10B, since the size of the reference tooth 830 is smaller than that of the target tooth 400, the ratio at which the shape of the reference tooth 830 is exposed is low. If the shape of the prosthesis is generated on the basis of the shape of the reference tooth 830, the prosthesis may be generated with a shape that is not suitable for the oral structure of the subject. As such, it is necessary to appropriately adjust the size of the reference tooth 830.

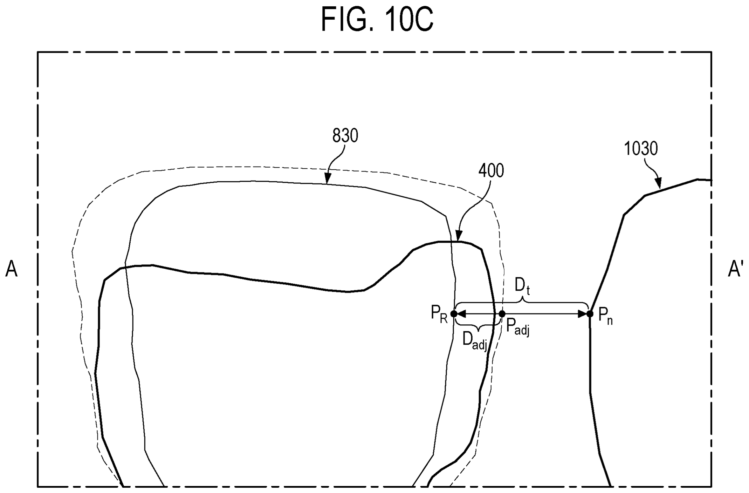

FIG. 10C is a diagram illustrating a process of adjusting the size of a reference tooth 830 for alignment between a target tooth 400 and the reference tooth 830 according to an embodiment of the present disclosure. FIG. 10C is a cross-sectional view taken along line A-A' of FIG. 10B.

According to an embodiment, the electronic device 100 may adjust the size of the reference tooth 830 on the basis of a distance between the reference tooth 830 and an adjacent tooth 1030 of the target tooth 400.

For example, the electronic device 100 may select a specific point P_{R} from among multiple points configuring an outline of the reference tooth 830. The electronic device 100 may determine a point Pₙ closest to the selected point P_{R} from among multiple points configuring the outline of the adjacent tooth 1030. The electronic device 100 may determine a specific point P_{adj} on a straight line connecting the point P_{R} and the point Pn. Here, the point P_{adj} may be a point spaced a specific distance D_{adj} apart from the point Pᵣ. The specific distance D_{adj} may be shorter than the total distance Dₜ between the point P_{adj} and the point Pᵣ. Subsequently, the electronic device 100 may adjust the size of the reference tooth 830 so that the point P_{R} moves to a position of the point P_{adj}. That is, the size of the reference tooth 830 may be adjusted by allowing the point P_{adj} to define the outline of the reference tooth 830.

For example, the electronic device 100 may adjust the size of the reference tooth 830 by a scaling factor of α. Here, when α is a value greater than or equal to 0 and less than 1, the size of the reference tooth 830 may be adjusted to be smaller than the existing size. Here, when α is a value greater than 1, the size of the reference tooth 830 may be adjusted to be larger than the existing size. For example, the electronic device 100 may calculate a transformation matrix that adjusts the size of the reference tooth 830 by a factor of α. The transformation matrix may be a matrix including one or more basis vectors that move a corresponding point in a direction of a normal vector at a point on the outline of the reference tooth 830. The electronic device 100 may multiply each of multiple points configuring the outline of the reference tooth 830 by the transformation matrix. The multiple points configuring the outline of the reference tooth 830 are moved to multiple specific points by the transformation matrix, and the multiple specific points configure the outline of the reference tooth 830, thereby adjusting the size of the reference tooth 830. For example, the electronic device 100 may multiply the point P_{R} configuring the outline of the reference tooth 830 by the transformation matrix. Accordingly, the point P_{R} may be moved to the point P_{adj}. For example, the electronic device 100 may adjust the size of the reference tooth 830 through the transformation matrix within a range where the reference tooth 830 does not contact the adjacent tooth 1030.

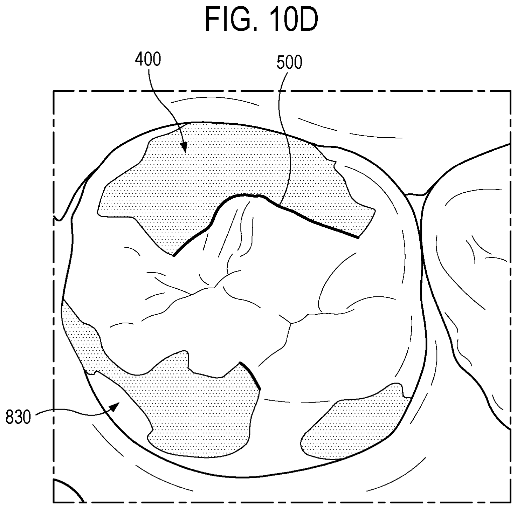

FIG. 10D is a diagram illustrating a result of adjusting the size of a reference tooth 830 for alignment between a target tooth 400 and the reference tooth 830 according to an embodiment of the present disclosure. Referring to FIG. 10D, the ratio of the shape of the reference tooth 830 that is exposed to the outside has increased compared to FIG. 10B before the size of the reference tooth 830 is corrected. When the shape of the prosthesis is generated on the basis of the shape of the reference tooth 830 as shown in FIG. 10D, the prosthesis may be generated to have a shape suitable for the oral structure of the subject.

Meanwhile, in FIGS. 10B and 10C, the operation of the electronic device 100 adjusting the orientation and size of the reference tooth 830 for alignment with the target tooth 400 may be expressed as an operation of generating data for the adjustment of the orientation and size of the reference tooth 830. For example, the data for the adjustment of the orientation and size of the reference tooth 830 may include the common local coordinate system 1000 shared by the target tooth 400 and the reference tooth 830, the size of the reference tooth 830, the distance between the reference tooth 830 and the adjacent tooth 1030 of the target tooth 400, and the like. For example, the data for the adjustment of the orientation and size of the reference tooth 830 may represent an image of the reference tooth 830 aligned with the target tooth 400 according to the result of the adjustment of the orientation and size of the reference tooth 830.

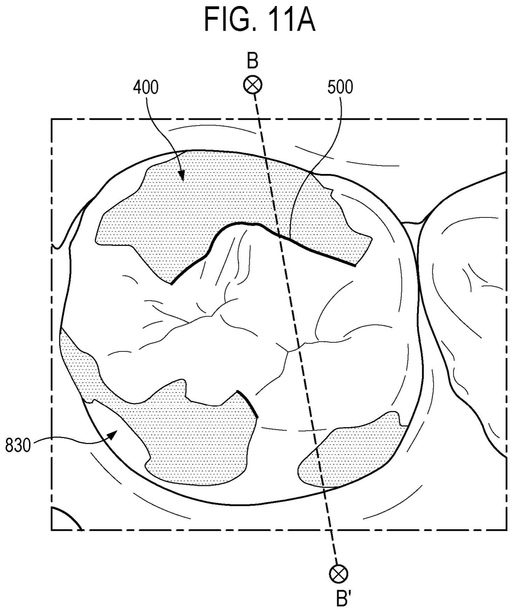

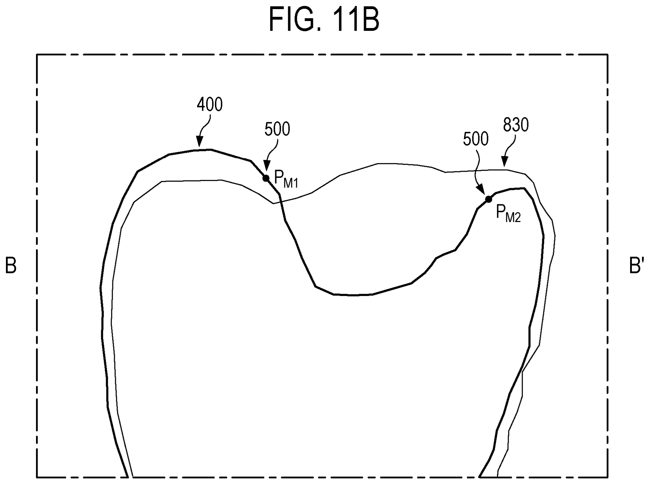

FIG. 11A is diagram illustrating a target tooth 400 aligned with a reference tooth 830 according to an embodiment of the present disclosure. FIG. 11B is a cross-sectional view taken along line B-B' of FIG. 11A.

Referring to FIG. 11A, since the orientation and size of the reference tooth 830 are adjusted so that the reference tooth 830 and the target tooth 400 are aligned, the electronic device 100 may generate inner and outer faces of the prosthesis on the basis of the margin line 500 of the target tooth 400 and the shape of the reference tooth 830. Here, the inner face of the prosthesis may be a surface of the prosthesis that comes into contact with a resin material layer inserted on a surface surrounded by the margin line 500 among surfaces of the target tooth 400. In other words, the inner face of the prosthesis will be arranged to face the surface of the target tooth 400 when the prosthesis is actually inserted into the oral cavity. In addition, the outer face of the prosthesis may be a surface of the prosthesis that configures the outline of the prosthesis when the prosthesis is attached to the target tooth 400. In other words, the outer face of the prosthesis is a portion that is exposed after the prosthesis is actually inserted into the oral cavity.

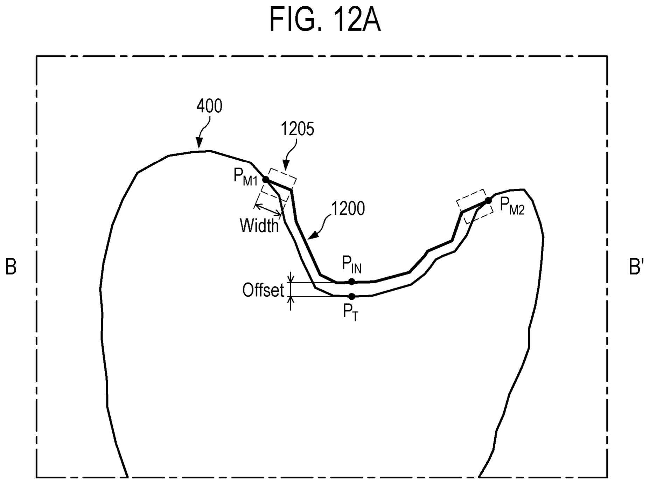

FIG. 12A is a diagram illustrating a process of generating an inner face 1200 of a prosthesis according to an embodiment of the present disclosure.

According to an embodiment, the electronic device 100 may determine a target point P_{T} surrounded by the margin line 500 among multiple points configuring a surface of the target tooth 400. The electronic device 100 may determine a normal direction with respect to the surface of the target tooth 400 at the target point P_{T}. Thereafter, the electronic device 100 may determine a point P_{IN} spaced a first offset apart from the target point P_{T} in the normal direction. Here, the first offset may be a predetermined value. Alternatively, the electronic device 100 may receive an input for the first offset from the user 10 through the input device 109. For example, the first offset may be a thickness of a resin material layer inserted under an inner face 1200. As a specific example, the first offset may be 0.1 mm. As such, the electronic device 100 may determine multiple points P_{IN} with respect to multiple target points P_{T} surrounded by the margin line 500 among multiple points configuring the surface of the target tooth 400. The electronic device 100 may generate the inner face 1200 of the prosthesis on the basis of the multiple points P_{IN}. That is, the inner face 1200 of the prosthesis may include the multiple points P_{IN}. The operation of generating the inner face 1200 of the prosthesis may be expressed as an operation of generating data for the inner face 1200 of the prosthesis. In this case, the data for the inner face 1200 of the prosthesis may include the multiple points P_{IN}.

According to an embodiment, a boundary region 1205 may be configured in a peripheral area of the margin line 500. For example, the boundary region 1205 may be determined in advance by a curvature of the target tooth 400 in a peripheral area of the margin line 500. Alternatively, the boundary region 1205 may be configured by the user 10. For example, the electronic device 100 may receive an input for the boundary region 1205 from the user 10 through the input device 109. For example, a margin parameter may be configured in the boundary region 1205. Here, the margin parameter may include the width and the angle of the boundary region 1205.

For example, the electronic device 100 may determine a target point P'_{T} located in the boundary region 1205 among multiple target points P_{T}. The electronic device 100 may apply a second offset to the target point P'_{T} instead of applying the first offset. That is, the electronic device 100 may determine a normal direction for a surface of the target tooth 400 at the target point P'_{T} and determine an offset point P'_{IN} spaced apart from the target point P'_{T} in the normal direction by the second offset instead of the first offset. Here, the second offset may be an offset determined such that an angle formed between a straight line connecting the point P_{M1} on the margin line 500 and the target point P'_{T} and a straight line connecting the point P_{M1} and the offset point P'_{IN} becomes an angle included in a margin parameter of the boundary region 1205. Accordingly, the closer the target point P'_{T} is to the margin line 500 within the boundary region 1205, the smaller the offset applied to the corresponding target point P'_{T}, and eventually, the target point P'_{T} located at an end of the boundary region 1205 meets the margin line 500. In addition, by configuring the boundary region 1205, the thickness of the resin material layer may become thinner as it approaches the margin line 500.

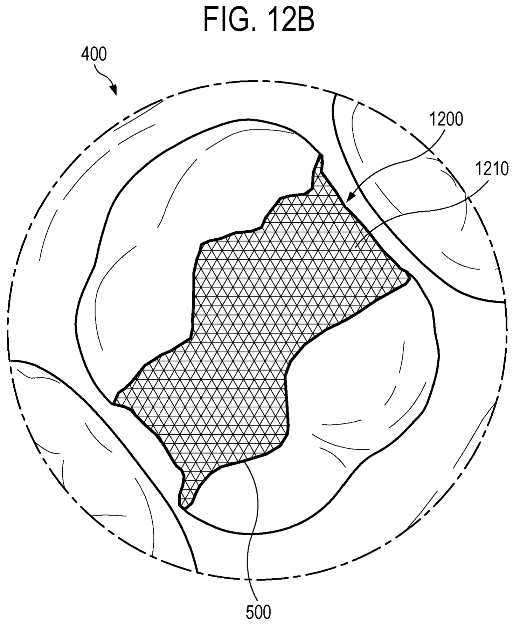

FIG. 12B is a diagram illustrating a mesh for an inner face 1200 of a prosthesis according to an embodiment of the present disclosure.

As described in FIG. 12A, the electronic device 100 may generate the inner face 1200 of the prosthesis. The operation of generating the inner face 1200 of the prosthesis may be expressed as an operation of generating data for the inner face 1200 of the prosthesis. Here, the data for the inner face 1200 of the prosthesis may be mesh data for the shape of the inner face 1200. For example, the mesh data for the shape of the inner face 1200 may include multiple faces 1210 representing the shape of the inner face 1200. For example, the multiple faces 1310 may be defined by multiple points configuring the inner face 1200 of the prosthesis. Meanwhile, FIG. 12B illustrates a triangular face 1210, but the present disclosure is not limited thereto. The face 1210 may be a square or a polygon. In addition, the number or size of the faces 1210 may be determined in advance.

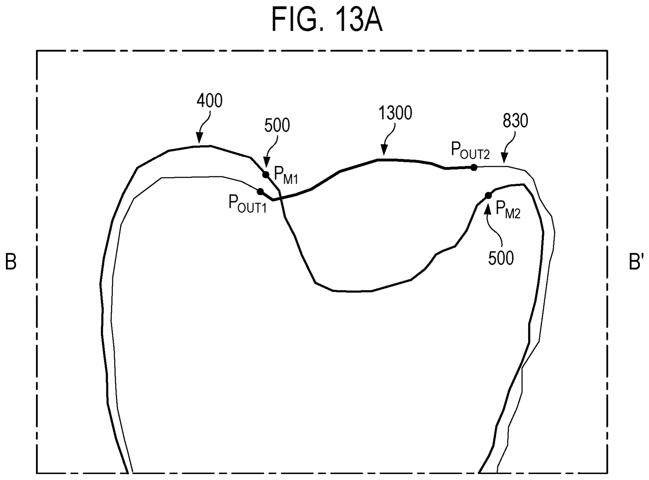

FIG. 13A is a diagram illustrating a process of generating an initial outer face 1300 of a prosthesis according to an embodiment of the present disclosure.

According to an embodiment, the electronic device 100 may generate an initial outer face 1300 of the prosthesis on the basis of the shape of the reference tooth 830 aligned with the margin line 500 and the target tooth 400.

For example, the electronic device 100 may determine multiple initial points P_{OUT1} and P_{OUT2} configuring a closed curve from among multiple points configuring the outline of the reference tooth 830. For example, the electronic device 100 may determine a point P_{OUT1} or P_{OUT2} on the outline of the reference tooth 830 that is closest to the points P_{M1} or P_{M2} configuring the margin line 500 as the initial point. Referring to the embodiment of FIG. 13A, the electronic device 100 may determine a point P_{OUT1} on the outline of the reference tooth 830 that is closest to the point P_{M1} on the margin line 500 as the initial point. In addition, the electronic device 100 may determine a point P_{OUT2} on the outline of the reference tooth 830 that is closest to the point P_{M2} on the margin line 500 as the initial point. As such, the electronic device 100 may perform the operation for at least some of the multiple points configuring the margin line 500 to determine multiple initial points configuring the closed curve.

For example, the electronic device 100 may generate the initial outer face 1300 of the prosthesis on the basis of the multiple initial points. That is, the initial outer face 1300 of the prosthesis may include the multiple initial points P_{OUT1} and P_{OUT2}. Meanwhile, the operation of generating the initial outer face 1300 of the prosthesis may be expressed as an operation of generating data for the initial outer face 1300 of the prosthesis. In this case, the data for the initial outer face 1300 of the prosthesis may include the multiple initial points P_{OUT1} and P_{OUT2}.

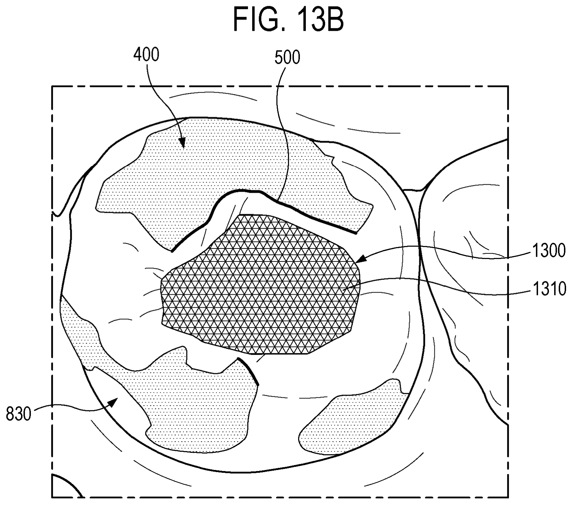

FIG. 13B is a diagram illustrating a mesh for an initial outer face 1300 of a prosthesis according to an embodiment of the present disclosure.

As described in FIG. 13A, the electronic device 100 may generate data for the initial outer face 1300 of the prosthesis. Here, the data for the initial outer face 1300 of the prosthesis may be mesh data for the shape of the initial outer face 1300. For example, the mesh data for the shape of the outer face 1300 may include multiple faces 1310 representing the shape of the initial outer face 1300. For example, the multiple faces 1310 may be defined by multiple points configuring the initial outer face 1300 of the prosthesis. Meanwhile, FIG. 13B illustrates a triangular face 1310, but the present disclosure is not limited thereto. The face 1310 may be a square or a polygon. In addition, the number or size of the faces 1310 may be determined in advance.

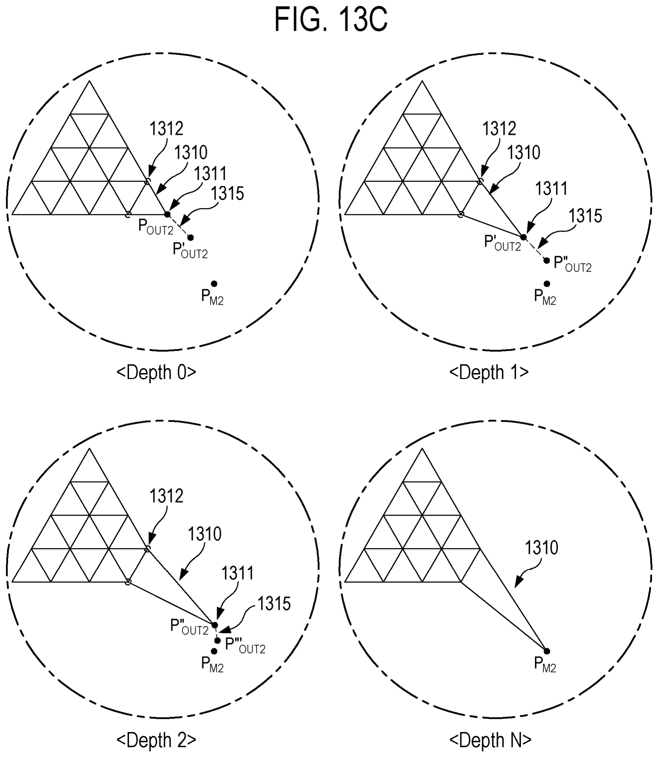

FIG. 13C is a diagram illustrating a process of modifying an outer face of a prosthesis according to an embodiment of the present disclosure.

As shown in FIGS. 13A and 13B, an edge of the initial outer face 1300 of the prosthesis may not be in contact with the margin line 500. Therefore, it is necessary to modify the outer face of the prosthesis so that the edge of the outer face of the prosthesis is in contact with the margin line 500 to define a boundary line between the outer face of the prosthesis and the margin line 500. To this end, the electronic device 100 may modify the outer face of the prosthesis. Meanwhile, the operation of modifying the outer face of the prosthesis may be expressed as an operation of generating data for the modified outer face of the prosthesis.

According to an embodiment, the electronic device 100 may move multiple points configuring the edge of an outer face of the prosthesis in the direction of the margin line 500 so that the edge of the outer face of the prosthesis comes into contact with the margin line 500. For example, the electronic device 100 may determine a movement path in a direction in which the margin line 500 is located with respect to each of the multiple points configuring the edge of the outer face of the prosthesis, and may move the multiple points configuring the edge of the outer face of the prosthesis along the determined movement path. That is, the electronic device 100 may determine the movement path in the direction in which the margin line 500 is located with respect to each of the points configuring the edge of the outer face of the prosthesis, and may modify the outer face of the prosthesis by interpolating the points on the determined movement path. Here, the electronic device 100 may modify the outer face of the prosthesis on the basis of radial basis function (RBF) interpolation. The electronic device 100 may modify the outer face of the prosthesis so that all points configuring the edge of the outer face of the prosthesis are located on the margin line 500 by repeatedly performing a point interpolation operation of determining the movement path for each of the points configuring the edge of the outer face of the prosthesis and moving the points.

Referring to the example of FIG. 13C, a process in which the electronic device 100 interpolates points on the basis of RBF interpolation to modify the outer face of the prosthesis will be described. FIG. 13C is an enlarged diagram illustrating a surrounding area of the point P_{OUT2} in FIG. 13A.

When a depth is 0, the electronic device 100 may determine a movement target point P_{OUT2} among the multiple points configuring the edge of the outer face of the prosthesis. Here, the depth may be the number of times the electronic device 100 performs the interpolation operation to determine (generate) the movement target point with respect to all points configuring the edge of the outer face of the prosthesis. That is, when the electronic device 100 performs the interpolation operation with respect to all points configuring the edge of the outer face of the prosthesis, the depth may increase by 1. For example, the electronic device 100 may determine a target face including the movement target point P_{OUT2} among the multiple faces 1310 configuring the shape of the outer face of the prosthesis. For example, the electronic device 100 may determine the movement target point P_{OUT2} as a control point 1311 that may move with respect to the target face. For example, the electronic device 100 may determine the remaining points, excluding the moving target point P_{OUT2}, among the multiple points configuring the target face as fixed points 1312 whose positions are fixed even if the moving target point P_{OUT2} moves. For example, the electronic device 100 may determine a movement path 1315 for the moving target point P_{OUT2}. For example, the electronic device 100 may determine a path along which the moving target point P_{OUT2} moves to an offset point P'_{OUT2} that is spaced a third offset apart in a direction with the shortest distance from the moving target point P_{OUT2} to the margin line 500 or the point P_{M2} on the margin line 500, as the movement path 1315 for the moving target point P_{OUT2}. Here, the third offset may be a predetermined value. Alternatively, the electronic device 100 may receive an input for the third offset from the user 10 through the input device 109. For example, the electronic device 100 may move the movement target point P_{OUT2} to the offset point P'_{OUT2} along the movement path 1315. Here, the operation of moving the movement target point P_{OUT2} to the offset point P'_{OUT2} may also be expressed as an operation of interpolating the offset point P'_{OUT2}.

When a depth is 1, the electronic device 100 may determine a movement target point P'_{OUT2} among the multiple points configuring the edge of the outer face of the prosthesis. For example, the electronic device 100 may determine a target face including the movement target point P'_{OUT2} among the multiple faces 1310 configuring the shape of the outer face of the prosthesis. For example, the electronic device 100 may determine the movement target point P'_{OUT2} as a control point 1311 that may move with respect to the target face. For example, the electronic device 100 may determine the remaining points, excluding the moving target point P'_{OUT2}, among the multiple points configuring the target face as fixed points 1312 whose positions are fixed even if the moving target point P'_{OUT2} moves. For example, the electronic device 100 may determine a movement path 1315 for the moving target point P'_{OUT2}. For example, the electronic device 100 may determine a path along which the moving target point P'_{OUT2} moves to an offset point P"_{OUT2} that is spaced the third offset apart in a direction with the shortest distance from the moving target point P'_{OUT2} to the margin line 500 or the point P_{M2} on the margin line 500, as the movement path 1315 for the moving target point P'_{OUT2}. For example, the electronic device 100 may move the movement target point P'_{OUT2} to the offset point P"_{OUT2} along the movement path 1315. Here, the operation of moving the movement target point P'_{OUT2} to the offset point P"_{OUT2} may also be expressed as an operation of interpolating the offset point P"_{OUT2}.

When a depth is 2, the electronic device 100 may determine a movement target point P"_{OUT2} among the points configuring the edge of the outer face of the prosthesis. For example, the electronic device 100 may determine a target face including the movement target point P"_{OUT2} among the multiple faces 1310 configuring the shape of the outer face of the prosthesis. For example, the electronic device 100 may determine the movement target point P"_{OUT2} as a control point 1311 that may move with respect to the target face. For example, the electronic device 100 may determine the remaining points, excluding the moving target point P"_{OUT2}, among the multiple points configuring the target face as fixed points 1312 whose positions are fixed even if the moving target point P"_{OUT2} moves. For example, the electronic device 100 may determine a movement path 1315 for the moving target point P"_{OUT2}.For example, the electronic device 100 may determine a path along which the moving target point P"_{OUT2} moves to an offset point P‴_{OUT2} that is spaced the third offset apart in a direction with the shortest distance from the moving target point P"_{OUT2} to the margin line 500 or the point P_{M2} on the margin line 500, as the movement path 1315 for the moving target point P"_{OUT2}. For example, the electronic device 100 may move the movement target point P"_{OUT2} to the offset point P‴_{OUT2} along the movement path 1315. Here, the operation of moving the movement target point P"_{OUT2} to the offset point P‴_{OUT2} may also be expressed as an operation of interpolating the offset point P‴_{OUT2}.

The electronic device 100 may repeatedly perform the point interpolation operation to modify the outer face of the prosthesis so that, when the depth is N (a natural number), the points configuring an outermost part of the prosthesis may be located on the margin line 500.

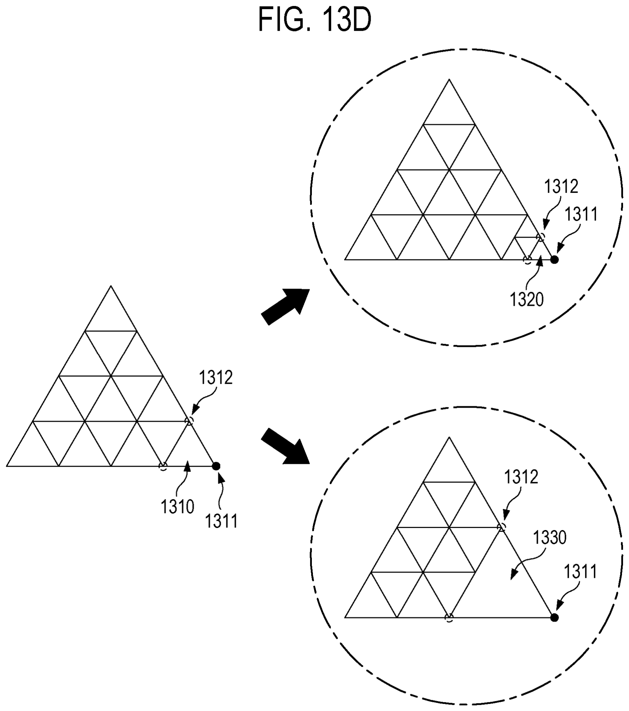

FIG. 13D is a diagram illustrating a process of adjusting a density of a mesh for an outer face of a prosthesis when modifying the outer face according to an embodiment of the present disclosure.

As described in FIG. 13C, in a process in which the electronic device 100 performs the point interpolation operation so that the points configuring the edge of the outer face of the prosthesis may be located on the margin line 500, the size of some of the multiple faces 1310 configuring the shape of the outer face of the prosthesis may change.

According to an embodiment, the electronic device 100 may determine whether a size of a specific face among the multiple faces 1310 defining the shape of the outer face of the prosthesis is greater than or equal to a predetermined size. Here, the specific face may be a face located at an edge of the outer face of the prosthesis among the multiple faces 1310.

According to an embodiment, when the size of the specific face is equal to or greater than the predetermined size, the electronic device 100 may divide the specific face into multiple sub-faces 1320. In this case, some of the points configuring one sub-face 1320 may be determined as control points 1311, and some other points may be determined as fixed points 1312.

According to an embodiment, when the size of the specific face is less than the predetermined size, the electronic device 100 may merge one or more adjacent faces of the specific face among the multiple faces 1310 to generate a merged face 1330. In this case, some of the points configuring the merged face 1330 may be determined as control points 1311, and some other points may be determined as fixed points 1312.

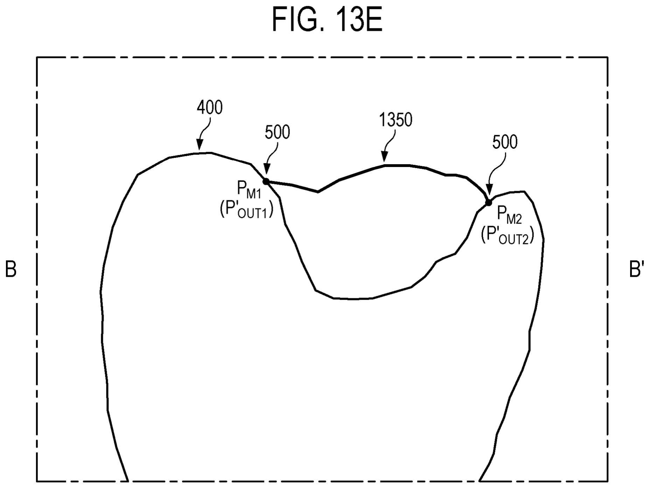

FIG. 13E is a diagram illustrating a modified outer face 1350 of a prosthesis according to an embodiment of the present disclosure.

According to an embodiment, the electronic device 100 may generate a modified outer face 1350 of the prosthesis through the interpolation operation described in FIG. 13C. Multiple points P'_{OUT1} and P'_{OUT2} configuring an edge of the modified outer face 1350 of the prosthesis may be located on the margin line 500. Referring to the embodiment of FIG. 13E, the point P'_{OUT1} configuring the edge of the modified outer face 1350 of the prosthesis may be identical to the point P_{M1} on the margin line 500. In addition, the point P'_{OUT2} configuring the edge of the modified outer face 1350 of the prosthesis may be identical to the point P_{M2} on the margin line 500.

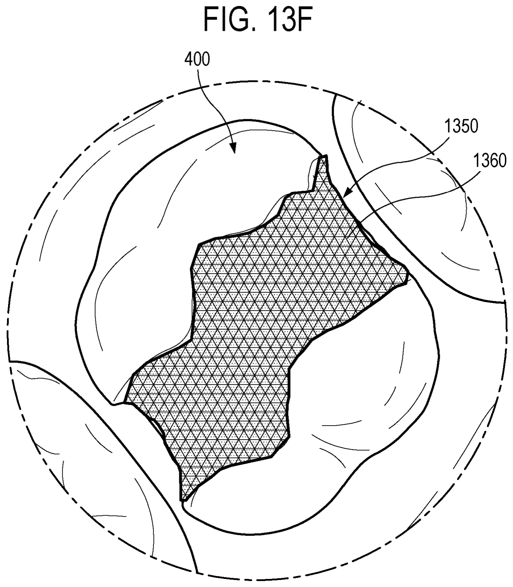

FIG. 13F is a diagram illustrating a mesh for a modified outer face 1350 of a prosthesis according to an embodiment of the present disclosure.

According to an embodiment, the electronic device 100 may generate data for the modified outer face 1350 of the prosthesis. Here, the data for the modified outer face 1350 of the prosthesis may be mesh data for the shape of the modified outer face 1350. For example, the mesh data for the shape of the modified outer face 1350 may include multiple faces 1360 representing the shape of the modified outer face 1350. For example, the multiple faces 1360 may be defined by multiple points configuring the modified outer face 1350 of the prosthesis. Meanwhile, FIG. 13F illustrates a triangular face 1360, but the present disclosure is not limited thereto. The face 1360 may be a square or a polygon. In addition, the number or size of the faces 1360 may be determined in advance.

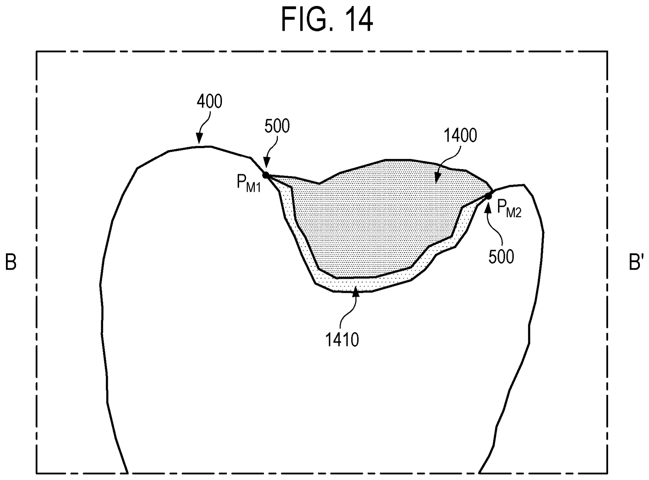

FIG. 14 is a diagram illustrating a shape of a prosthesis 1400 according to an embodiment of the present disclosure.

According to an embodiment, the electronic device 100 may generate the prosthesis 1400 on the basis of the inner face 1200 and the outer face 1350. The operation of generating the prosthesis 1400 may be expressed as an operation of generating data for the shape of the prosthesis 1400. Here, the data for the shape of the prosthesis 1400 may include multiple points configuring the shape of the prosthesis 1400.

For example, the electronic device 100 may generate the prosthesis 1400 by connecting multiple points configuring the edge of the inner face 1200 and multiple points configuring the edge of the outer face 1350.

According to an embodiment, the prosthesis 1400 may be attached to the target tooth 400 along the margin line 500.

According to an embodiment, a resin material layer 1410 may be inserted between the prosthesis 1400 and the target tooth 400.

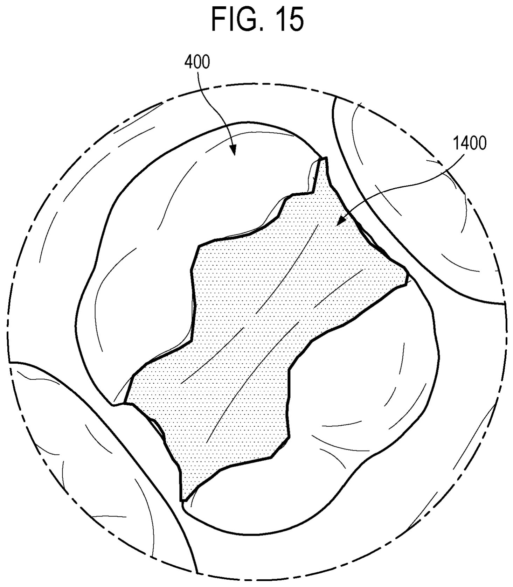

FIG. 15 is a diagram illustrating the shape of a prosthesis 1400 attached to a target tooth 400 according to an embodiment of the present disclosure.

Referring to FIG. 15, a boundary between the prosthesis 1400 and the target tooth 400 may be defined by the margin line 500.

Meanwhile, although the embodiment of the present disclosure has been described based on the mandible, the user 10 may select the maxillary teeth and the mandibular teeth as the target teeth 400. In this case, the electronic device 100 may perform the operation according to the above-described embodiment for each of the maxillary target teeth 400 and the mandibular target teeth 400 to generate a prosthesis 1400 to be attached to the maxillary target teeth 400 and a prosthesis 1400 to be attached to the mandibular target teeth 400. In case that the user 10 selects the maxillary teeth and the mandibular teeth in an antagonist tooth relationship as the target teeth 400, the electronic device 100 may generate the prosthesis 1400 for the maxilla and the prosthesis 1400 for the mandible, and determine which of the prostheses to adjust the shape thereof.

For example, the electronic device 100 may receive a selection input from the user 10 regarding a prosthesis to be adjusted among the prosthesis 1400 for the maxilla and the prosthesis 1400 for the mandible, and may determine which of the prosthesis 1400 for the maxilla and the prosthesis 1400 for the mandible to adjust the shape thereof on the basis of the selection input. For example, the electronic device 100 may determine to adjust the shape of the prosthesis 1400 that comes into contact with a greater number of prostheses 1400 attached to antagonist teeth. For example, with respect to the target teeth 400 of the maxilla and the target teeth 400 of the mandible in an antagonist tooth relationship, if the prosthesis 1400 attached to one of the target teeth 400 of the maxilla comes into contact with at least two of the prostheses 1400 attached to the target teeth 400 of the mandible, the electronic device 100 may determine to adjust the shape of the prosthesis 1400 of the maxilla. For example, with respect to the target teeth 400 of the maxilla and the target teeth 400 of the mandible in an antagonist tooth relationship, if the prosthesis 1400 attached to one of the target teeth 400 of the mandible comes into contact with at least two of the prostheses 1400 attached to the target teeth 400 of the maxilla, the electronic device 100 may determine to adjust the shape of the prosthesis 1400 of the mandible. For example, in response to the determination to adjust the shape of the prosthesis 1400 for the maxilla, the electronic device 100 may adjust the shape of the prosthesis 1400 for the maxilla on the basis of the shape of the prosthesis1400 for the mandible so that the prosthesis 1400 for the mandible and the prosthesis 1400 for the maxilla do not come into contact. Here, the adjusting of the shape may include cutting. For example, in response to the determination to adjust the shape of the prosthesis 1400 for the mandible, the electronic device 100 may adjust the shape of the prosthesis 1400 for the mandible on the basis of the shape of the prosthesis1400 for the maxilla so that the prosthesis 1400 for the maxilla and the prosthesis 1400 for the mandible do not come into contact. Meanwhile, the operation of adjusting the shape of the prosthesis 1400 may be expressed as an operation of modifying data for the shape of the prosthesis 1400 to represent the adjusted shape of the prosthesis 1400.

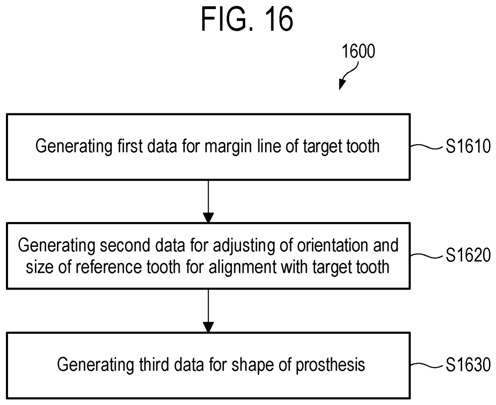

FIG. 16 is a flowchart illustrating a method 1600 according to an embodiment of the present disclosure. According to an embodiment, the method 1600 may be performed by the electronic device 100.

In S1610, the electronic device 100 may generate data ("first data") for the margin line 500 of the target tooth 400 included in the oral cavity on the basis of 3D scan data 300, 301, or 302 for the oral cavity of the subject 20. Here, the margin line 500 may be a closed curve defining a boundary between the prosthesis 1400 to be attached to the target tooth 400 and the target tooth 400. The operation of S1610 may refer to the description of FIG. 5.

According to an embodiment, the electronic device 100 may determine the target tooth 400 among one or more teeth included in the oral cavity of the subject 10 when generating the first data. For example, the electronic device 100 may receive a selection input for the target tooth 400 from the user 10 through the input device 109. The electronic device 100 may determine the target tooth 400 on the basis of the received selection input. This may refer to the description of FIG. 4A. Meanwhile, the electronic device 100 may also determine the target tooth 400 among one or more teeth included in the oral cavity of the subject 10 before S1610.

According to an embodiment, when generating the first data, the electronic device 100 may determine the type 410 of the prosthesis 1400 to be attached to the target tooth 400. For example, the electronic device 100 may receive a selection input for the type 410 of the prosthesis 1400 to be attached to the target tooth 400 from the user 10 through the input device 109. The electronic device 100 may determine the type 410 of the prosthesis 1400 to be attached to the target tooth 400 based on the received selection input. This may refer to the description of FIG. 4A. The electronic device 100 may also determine the type 410 of the prosthesis 1400 to be attached to the target tooth 400 before S1610.

In S1620, the electronic device 100 may generate data ("second data") for adjusting the orientation and size of the reference tooth 830 for alignment with the target tooth 400 on the basis of data for the dental library 800 including the reference tooth 830 corresponding to the target tooth 400. The operation of S1620 may refer to the description of FIGS. 8 to 10C.

According to an embodiment, when generating the second data, the electronic device 100 may generate data ("fourth data") for the arch curve 600 defining a maxillary or mandibular arch including the target tooth 400 on the basis of 3D scan data 300, 301, or 302. Thereafter, the electronic device 100 may adjust the orientation of the reference tooth 830 on the basis of the fourth data.

For example, the electronic device 100 may determine a local coordinate system 700 ("first local coordinate system 700") that defines the orientation of the target tooth 400 with respect to the arch curve 600 in adjusting the orientation of the reference tooth 830. The electronic device 100 may determine a local coordinate system 900 ("second local coordinate system (900)") that defines the orientation of the reference tooth 830 with respect to the maxillary or mandibular arch including the reference tooth 830 within the dental library 800. Thereafter, the electronic device 100 may adjust the orientation of the reference tooth 830 such that the axes 710 and 720 of the first local coordinate system 700 and the axes 910 and 920 of the second local coordinate system 900 are aligned.

For example, when determining the first local coordinate system 700, the electronic device 100 may determine a first direction 710 and a second direction 720 with respect to the arch curve 600 at a specific point among the points configuring the margin line 500. Thereafter, the electronic device 100 may determine the first local coordinate system 700 to include an axis of the first direction 710 and an axis of the second direction 720. For example, the first direction 710 may be a centripetal acceleration direction of the arch curve 600 at a specific point on the arch curve 600 that is closest to a specific point on the margin line 500. For example, the second direction 720 may be a tangential direction of the arch curve 600 at a specific point on the arch curve 600.

For example, data for the dental library 800 may include data for a local coordinate system for each of multiple model teeth included in the dental library 800. When determining the second local coordinate system 900, the electronic device 100 may determine a local coordinate system for the reference tooth 830 corresponding to the target tooth 400 among multiple model teeth in the dental library 800 as the second local coordinate system 900.

According to an embodiment, when generating the second data, the electronic device 100 may adjust the size of the reference tooth 830 on the basis of the distance between the reference tooth 830 whose orientation has been adjusted and the adjacent tooth 1030 of the target tooth 400.

In S1630, the electronic device 100 may generate data ("third data") on the shape of the prosthesis 1400 on the basis of the first data and the second data. The operation of S1630 may refer to the description of FIGS. 11A to 15.

According to an embodiment, when generating the third data, the electronic device 100 may connect one or more points configuring the inner face 1200 of the prosthesis and one or more points configuring the outer face 1350 of the prosthesis 1400.

According to an embodiment, the electronic device 100 may generate mesh data ("first mesh data") for the inner face 1200 of the prosthesis 1400. For example, the first mesh data may include multiple faces 1210 representing the shape of the inner face 1200 of the prosthesis 1400.

For example, when generating the first mesh data, the electronic device 100 may determine a normal direction for the surface of the target tooth 400 at a point configuring the margin line 500. The electronic device 100 may determine a first offset point spaced a first offset apart from the point in the normal direction. Thereafter, the electronic device 100 may generate the first mesh data for the basis of the first offset point.

According to an embodiment, the electronic device 100 may generate mesh data ("second mesh data") for the outer face 1350 of the prosthesis 1400. For example, the second mesh data may include multiple faces 1360 representing the shape of the outer face 1350 of the prosthesis 1400.

For example, when generating the second mesh data, the electronic device 100 may determine multiple initial points configuring a closed curve from among multiple points configuring an outline of the reference tooth 830 aligned with the target tooth 400. Thereafter, the electronic device 100 may generate mesh data ("third mesh data") for the initial outer face 1300 on the basis of the multiple initial points and the shape of the reference tooth 830. For example, the third mesh data may include multiple faces 1310 representing the shape of the initial outer face 1300. The electronic device 100 may generate the second mesh data for the outer face 1350 of the prosthesis 1400 by modifying the initial outer face 1300 so that the points configuring the edges of the initial outer face 1300 may be located on the margin line 500.

Although process operations, method operations, algorithms or the like may be described in a sequential order, such processes, methods and algorithms may be configured to work in any suitable order. In other words, any sequence or order of operations that may be described in the present disclosure does not, in and of itself, indicate a requirement that the operations be performed in that order. Further, some operations may be performed simultaneously in other embodiments despite being described or implied as occurring non-simultaneously. Moreover, the illustration of a process by its depiction in a drawing does not imply that the illustrated process is exclusive of other variations and modifications thereto, does not imply that the illustrated process or any of its operations are necessary to one or more embodiments of the present disclosure, and does not imply that the illustrated process is preferred.

While the foregoing methods have been described with respect to particular embodiments, these methods may also be implemented as computer-readable codes on a computer-readable recording medium. The computer-readable recording medium includes any kind of data storage devices that can be read by a computer system. Examples of the computer-readable recording medium includes a ROM, a RAM, a CD-ROM, a magnetic tape, a floppy disk, an optical data storage device, and the like. Also, the computer-readable recording medium can be distributed to the computer systems which are connected through a network so that the computer-readable codes can be stored and executed in a distributed manner. Further, the functional programs, codes and code segments for implementing the foregoing embodiments can easily be inferred by programmers in the art to which the present disclosure pertains.

## Claims

1. A method performed by an electronic device comprising at least one processor and at least one memory in which instructions executed by the at least one processor are stored, the method comprising:
generating first data for a margin line of a target tooth included in an oral cavity, based on three-dimensional scan data for the oral cavity of a subject, wherein the margin line corresponds to a closed curve defining a boundary between a prosthesis to be attached to the target tooth and the target tooth;
generating second data for adjusting an orientation and a size of a reference tooth for alignment with the target tooth, based on data for a dental library including the reference tooth corresponding to the target tooth; and
generating third data for a shape of the prosthesis, based on the first data and the second data.

2. The method of Claim 1, wherein the generating the first data comprises determining the target tooth among one or more teeth included in the oral cavity of the subject.

3. The method of Claim 1, wherein the generating the second data comprises:
generating fourth data for an arch curve defining a maxillary or mandibular arch including the target tooth, based on the three-dimensional scan data; and
adjusting the orientation of the reference tooth, based on the fourth data.

4. The method of Claim 3, wherein the adjusting the orientation of the reference tooth comprises:
determining a first local coordinate system that defines an orientation of the target tooth with respect to the arch curve;
determining a second local coordinate system that defines the orientation of the reference tooth with respect to a maxillary or mandibular arch including the reference tooth within the dental library; and
adjusting the orientation of the reference tooth such that axes of the first local coordinate system and axes of the second local coordinate system are aligned.

5. The method of Claim 4, wherein the determining the first local coordinate system comprises:
determining a first direction and a second direction with respect to the arch curve at a first point among points configuring the margin line; and
determining the first local coordinate system to include an axis of the first direction and an axis of the second direction.

6. The method of Claim 5, wherein the first direction is a centripetal acceleration direction of the arch curve at a second point on the arch curve that is closest to the first point, and
wherein the second direction is a tangential direction of the arch curve at the second point.

7. The method of Claim 4, wherein the data for the dental library comprises data for a local coordinate system for each of multiple model teeth included in the dental library, and
wherein the determining the second local coordinate system comprises determining a local coordinate system for the reference tooth corresponding to the target tooth among the multiple model teeth as the second local coordinate system.

8. The method of Claim 3, wherein the generating the second data comprises adjusting the size of the reference tooth, based on a distance between the reference tooth whose orientation has been adjusted and an adjacent tooth of the target tooth.

9. The method of Claim 8, wherein the adjusting the size of the reference tooth comprises:
selecting a first point from among points constituting an outline of the reference tooth;
determining a second point having a shortest distance to the first point among points constituting an outline of the adjacent tooth;
determining a third point on a straight line connecting the first point and the second point; and
adjusting the size of the reference tooth such that the first point is moved to a position of the third point.

10. The method of Claim 1, wherein the generating the third data comprises:
generating first mesh data for an outer face of the prosthesis; and
generating second mesh data for an inner face of the prosthesis.

11. The method of Claim 10, wherein the generating the third data comprises connecting one or more points constituting the inner face of the prosthesis and one or more points constituting the outer face of the prosthesis.

12. The method of Claim 10, wherein the generating the first mesh data comprises:
determining a normal direction with respect to a surface of the target tooth at a point configuring the margin line;
determining a first offset point spaced a first offset apart from the point in the normal direction; and
generating the first mesh data, based on the first offset point.

13. The method of Claim 10, wherein the generating the second mesh data comprises:
determining multiple initial points constituting a closed curve among multiple points constituting an outline of the reference tooth aligned with the target tooth; and
generating third mesh data for an initial outer face of the prosthesis, based on the multiple initial points and a shape of the reference tooth.

14. The method of Claim 13, wherein the third mesh data comprises multiple faces formed by the multiple initial points.

15. The method of Claim 14, further comprising:
determining a moving target point located at an edge of the initial outer face of the prosthesis among the multiple initial points;
determining a target face including the moving target point among the multiple faces;
determining the moving target point as a control point for the target face;
determining a second offset point that is spaced a second offset apart in a direction with a shortest distance from the moving target point determined as the control point with respect to the margin line; and
changing the target point constituting the target face to the second offset point.

16. The method of Claim 14, further comprising:
determining whether a size of a specific face among the multiple faces is equal to or greater than a specific value;
in response to determining that the size of the specific face is equal to or greater than the specific value, dividing the specific face into multiple sub-faces; and
in response to determining that the size of the specific face is less than the specific value, merging the specific face with one or more adjacent faces with respect to the specific face among the multiple faces.

17. An electronic device comprising:
at least one processor; and
at least one memory in which instructions executed by the at least one processor are stored,
wherein the at least one processor is configured to, when the instructions are executed by the at least one processor, execute the method according to one of Claims 1 to 16.

18. A non-transitory computer-readable recording medium recording instructions that, when executed by at least one processor, cause the at least one processor to perform operations,
wherein the instructions are configured to cause the at least one processor to execute the method according to one of Claims 1 to 16.
